# EUROPEAN PATENT SPECIFICATION

(11) **EP 1 162 956 B1**
(45) Date of publication and mention of the grant of the patent: **08.06.2005**
(21) Application number: 00906091.4
(22) Date of filing: 23.02.2000
(51) Int. Cl.: A61K 9/70

(54) **COMPOSITIONS AND METHODS FOR IMPROVING INTEGRITY OF COMPROMISED BODY PASSAGEWAYS AND CAVITIES**
ZUSAMMENSETZUNGEN UND VERFAHREN ZUR VERBESSERUNG DER INTEGRITÄT VON ANGEGRIFFENEN KÖRPERPASSAGEWEGEN UND HÖHLEN
COMPOSITIONS ET METHODES POUR L'AMELIORATION DE L'INTEGRITE DE CAVITES ET DE PASSAGES CORPORELS AFFAIBLIS

(30) Priority: 23.02.1999 US 121424 P
(43) Date of publication of application: 19.12.2001
(73) Proprietor: ANGIOTECH INTERNATIONAL AG, 6300 Zug (CH); UNIVERSITY OF BRITISH COLUMBIA, Vancouver, British Columbia, V6T 1Z3 (CA)
(72) Inventor: SIGNORE, Pierre, E., Vancouver, Bristish Columbia V6K 1L5 (CA); MACHAN, Lindsay, S., Vancouver, British Columbia V6R 1A2 (CA)
(74) Representative: Gowshall, Jonathan Vallance
(86) International application number: PCT/CA2000/000175
(87) International publication number: WO 2000/050016

(56) References cited:
- WO-A-95/03036
- WO-A-97/33552
- WO-A-98/57671
- US-A- 5 716 981

## Description

### TECHNICAL FIELD

The present invention relates generally to compositions and methods for improving the integrity of body passageways or cavities following surgery or injury, and more specifically, to compositions comprising therapeutic agents which may be delivered to the external walls of body passageways or cavities for the purpose of strengthening the walls of the passageway or cavity.

### BACKGROUND OF THE INVENTION

There are many passageways within the body which allow the flow of essential materials. These include, for example, arteries and veins, the esophagus, stomach, small and large intestine, biliary tract, ureter, bladder, urethra, nasal passageways, trachea and other airways, and the male and female reproductive tract. Injury, various surgical procedures, or disease can result in the narrowing, weakening and/or obstruction of such body passageways, resulting in serious complications and/or even death.

Vascular disease can result in the narrowing, weakening and/or obstruction of body passageways. According to 1995 estimates (source - U.S. Heart and Stroke Foundation homepage), close to 60 million Americans have one or more forms of cardiovascular disease. These diseases claimed over 950,000 lives in the same year (41.5% of all deaths in the United States).

Since the late 1970s, arterial and venous catherizations have become increasingly common. A more aggressive approach to cardiac and vascular disease has resulted in an increased number of diagnostic and interventional procedures, including coronary and peripheral angiograms, thrombolytic therapy, various types of angioplasty and intravascular stent implantation. Balloon angioplasty (with or without stenting) is one of the most widely used treatments for vascular disease. In 1998, 1.2 million percutaneous transluminal coronary angioplasties were performed worldwide, 70% of which included stent insertion (Medical Data International, MedPro Month, November-December 1998). The site of sheath entry for these arterial and venous catherizations leave vascular punctures ranging from 2 mm (7 to 12 French for balloon angioplasty) to 9 mm (24 to 27 French for stent graft insertion).

The incidence of iatrogenic complications of venous and arterial access has reached epidemic proportions. In fact, these injuries represent the most common type of vascular trauma in most hospitals, exceeding even those due to gunshot and knife wounds.

The resultant complications depend on the site of vascular injury as well as the type of procedure that is being performed. In the past, arterial thrombosis was the most common complication following angiography. Today, expanding hematomas and pseudoaneurysms predominate, due primarily to large catheter sheaths, the use of thrombolytic agents and anticoagulants, and longer duration of catheter use.

In addition to being a complication of iatrogenic arterial and venous catheterization, pseudoaneurysms can also result from a variety of mechanisms, including infection, trauma and diverse complications of vascular surgery leading to anastomotic separation. All have in common the disruption of arterial continuity with the resultant leakage of blood into the surrounding fibrous tissue capsule. The capsule progressively enlarges due to the continuous arterial pressure, leading to the formation of a pseudoaneurysm.

Other diseases can also lead to abnormal wound healing or complications due to diminished body passageway or cavity wall integrity. Briefly, these include aneurysms (*i*.*e*., aortic and peripheral vascular), iatrogenic or pathologic cardiac rupture or dissection (*i*.*e*., due to tissue necrosis following myorcardial infarction or myocardial dilation), aortic dissection, vessel dissection during any vascular surgical procedure, prosthetic cardiac valve dehiscence, gastrointestinal (GI) passageway rupture (*e.g.*, ulcers, postoperative) and any surgical wound repair.

The existing treatments for the above diseases and conditions for the most part share the same limitations. The use of therapeutic agents have not resulted in the reversal of these conditions and whenever an intervention is used to treat the conditions, there is a risk to the patient as a result of the body's response to the intervention. The present invention provides compositions and methods suitable for treating the conditions and diseases that are generally discussed above. These compositions and methods address the problems associated with the existing procedures, offer significant advantages when compared to existing procedures, and in addition, provide other, related advantages.

### SUMMARY OF THE INVENTION

Briefly stated, the present invention relates generally to compositions for improving the integrity of body passageways or cavities following surgery, or injury, and more specifically, to either polymers alone or compositions comprising therapeutic agents that induce fibrosis (either with or without a polymer) which may be delivered to the external walls of body passageways or cavities for the purpose of strengthening the walls of the passageway or cavity, as defined in the accompanying claims.

A wide variety of therapeutic agents are disclosed, including for example microtubule stabilizing agents (e.g., paclitaxel, or analogues or derivatives thereof), fibrosis inducers, angiogenic factors, growth factors and cytokines and other factors involved in the wound healing or fibrosis cascade.

Within certain embodiments of the invention, the therapeutic agents may further comprise a carrier (either polymeric or non-polymeric), such as, for example, poly(ethylene-vinyl acetate), poly(urethane), copolymers of lactic acid and glycolic acid, poly (caprolactone), poly (lactic acid), copolymers of poly (lactic acid) and poly (caprolactone), gelatin, hyaluronic acid, collagen matrices and albumen.

The therapeutic agents may be utilized to treat or prevent a wide variety of conditions, including, for example, iatrogenic complications of arterial and venous catheterization, aortic dissection, cardiac rupture, aneurysm, pseudoaneurysms, cardiac valve dehiscence, passageway rupture, periprosthetic leaks and surgical wound repair. Representative body passageways and cavities that may be treated include, for example, arteries, veins, the heart, the esophagus, the stomach, the duodenum, the small intestine, the large intestine, biliary tracts, the biliary duct, the ureter, the bladder, the urethra, lacrimal ducts, the trachea, bronchi, bronchiole, nasal passages (including the sinuses) and other airways, eustachian tubes, the external auditory canal, the vas deferens and other passageways of the male reproductive tract, the uterus and fallopian tubes and the ventricular system (cerebrospinal fluid) of the brain and the spinal cord. Representative examples of cavities include, for example, the abdominal cavity, the buccal cavity, the peritoneal cavity, the pericardial cavity, the pelvic cavity, perivisceral cavity, pleural cavity, inguinal canal and uterine cavity.

Within one particularly preferred embodiment of the invention, the therapeutic agent is delivered to an artery or vein by direct injection into the adventia.

These and other aspects of the present invention will become evident upon reference to the following detailed description and attached drawings.

### BRIEF DESCRIPTION OF THE DRAWINGS

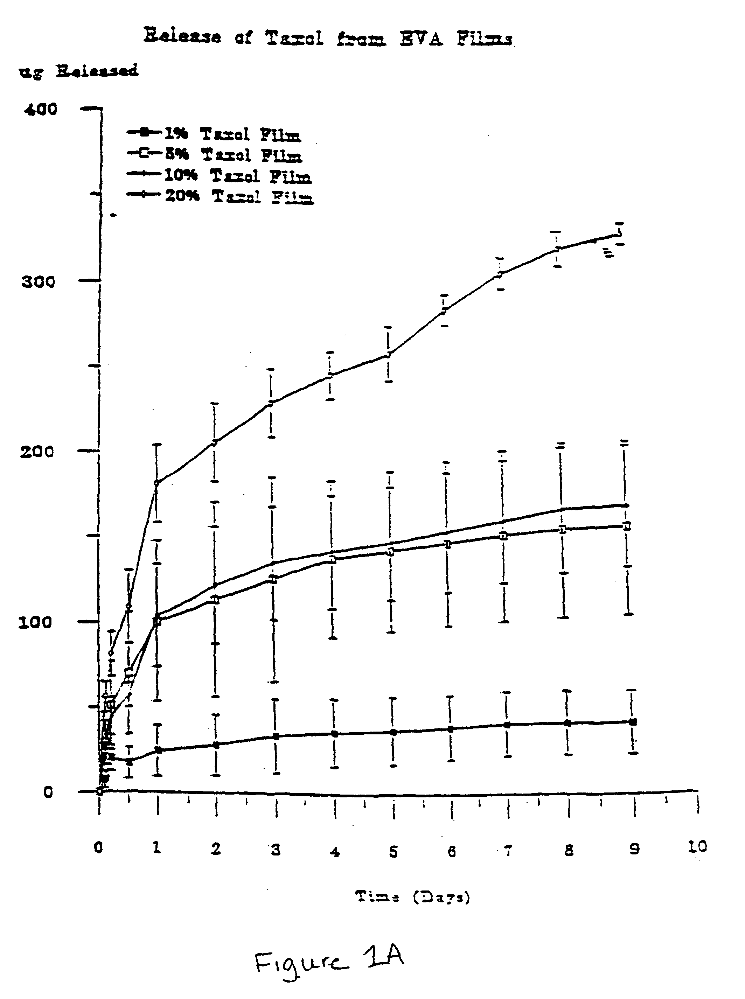

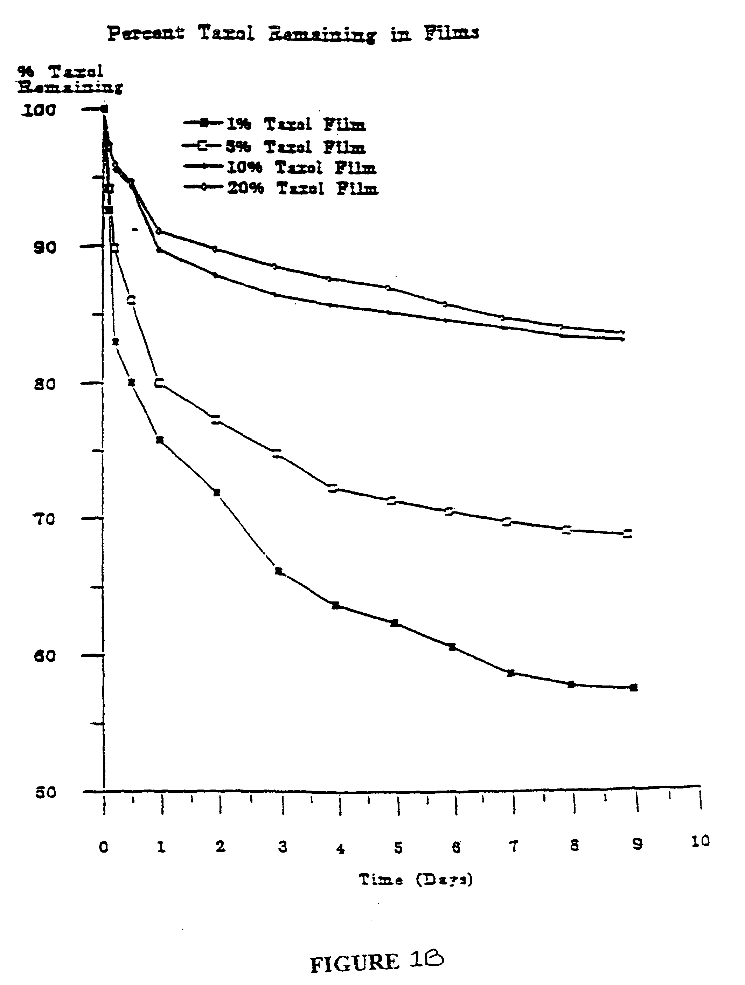

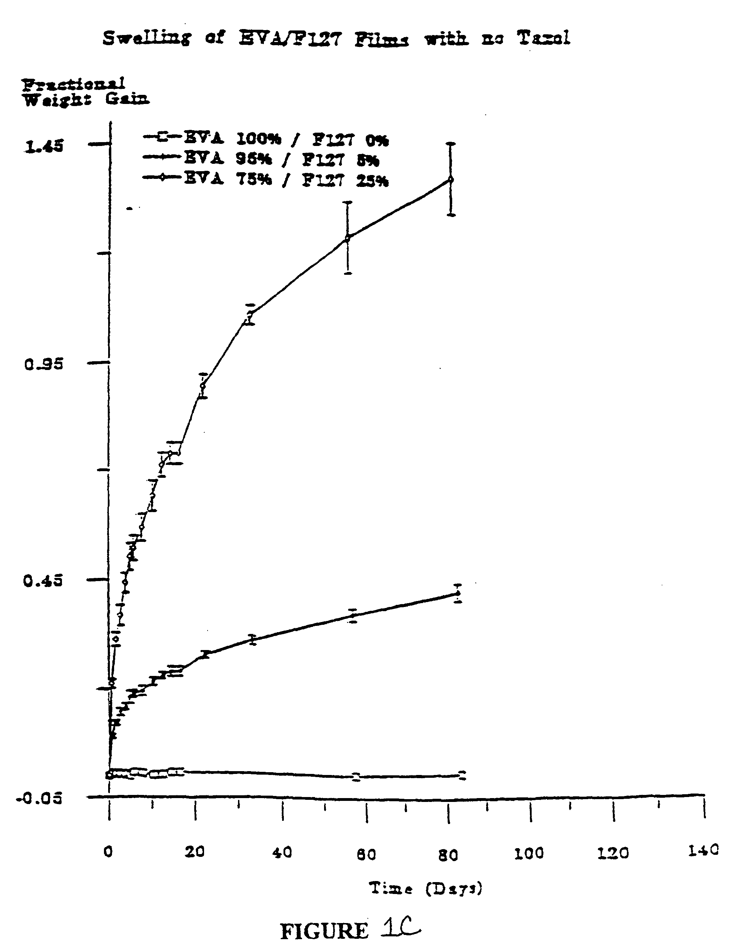

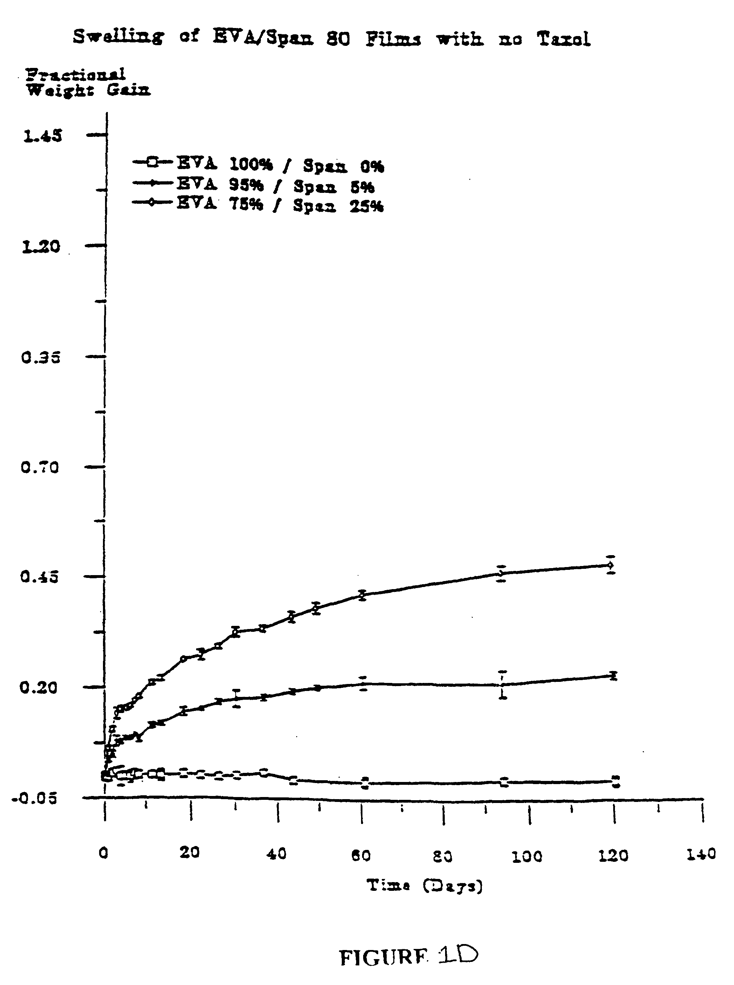

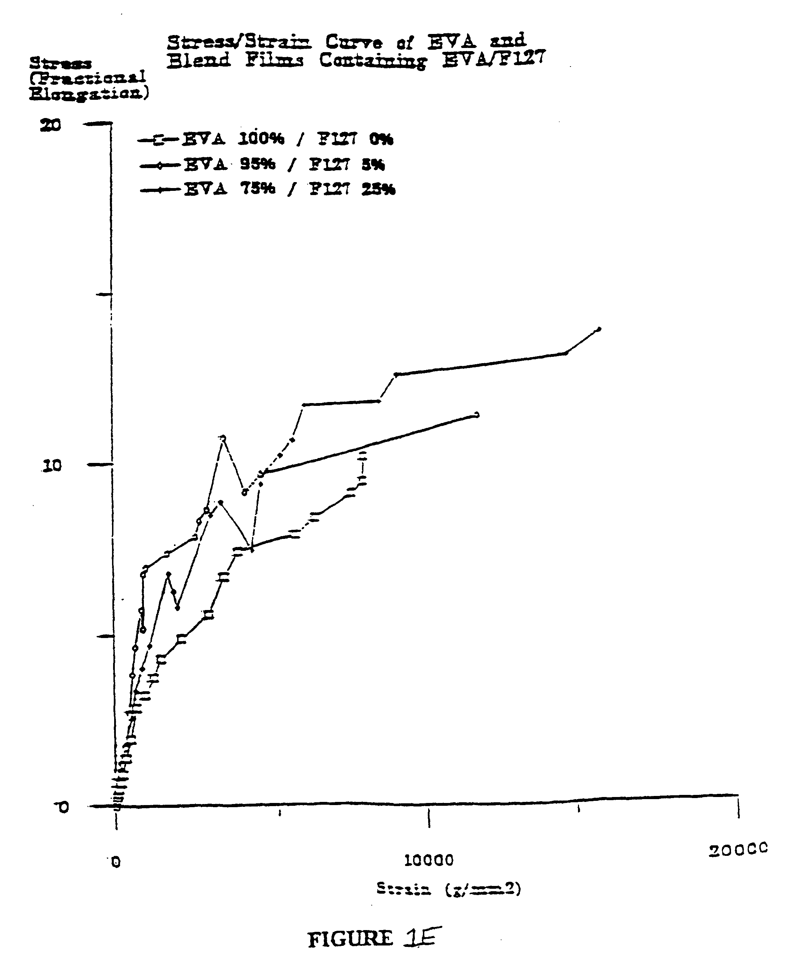

Figures 1A and 1B, respectively, are two graphs which show the release of paclitaxel from EVA films, and the percent paclitaxel remaining in those same films over time. Figure 1C is a graph which shows the swelling of EVA/F127 films with no paclitaxel over time. Figure 1D is a graph which shows the swelling of EVA/Span 80 films with no paclitaxel over time. Figure 1E is a graph which depicts a stress vs. strain curve for various EVA/F127 blends.

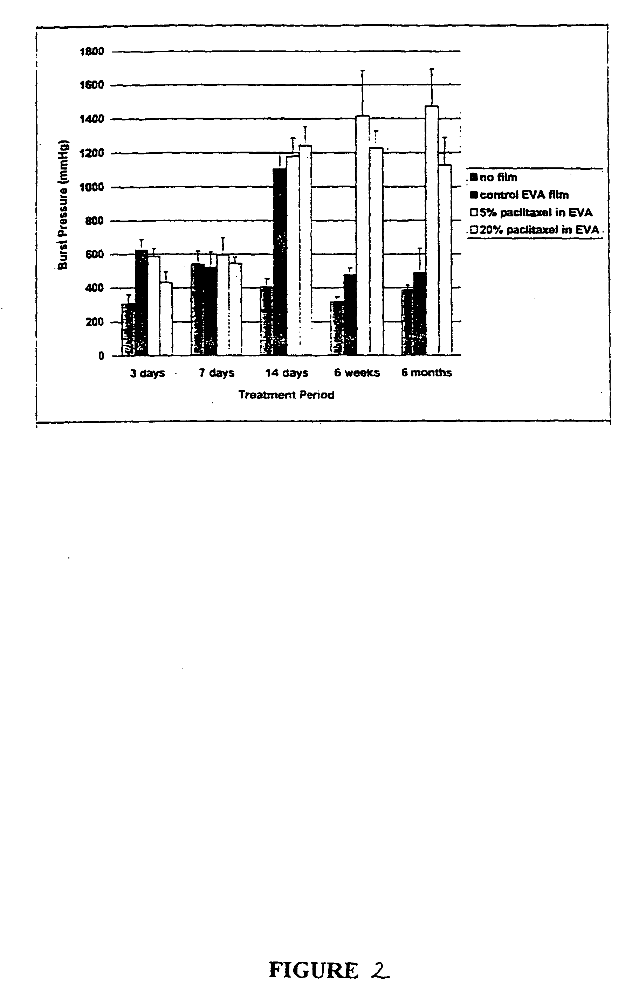

Figure 2 is a graph which shows burst pressure of aortic wounds treated with EVA films containing different concentrations of paclitaxel at 3 days, 7 days, 14 days, 6 weeks and 6 months after surgery and treatment (n=5 in each group).

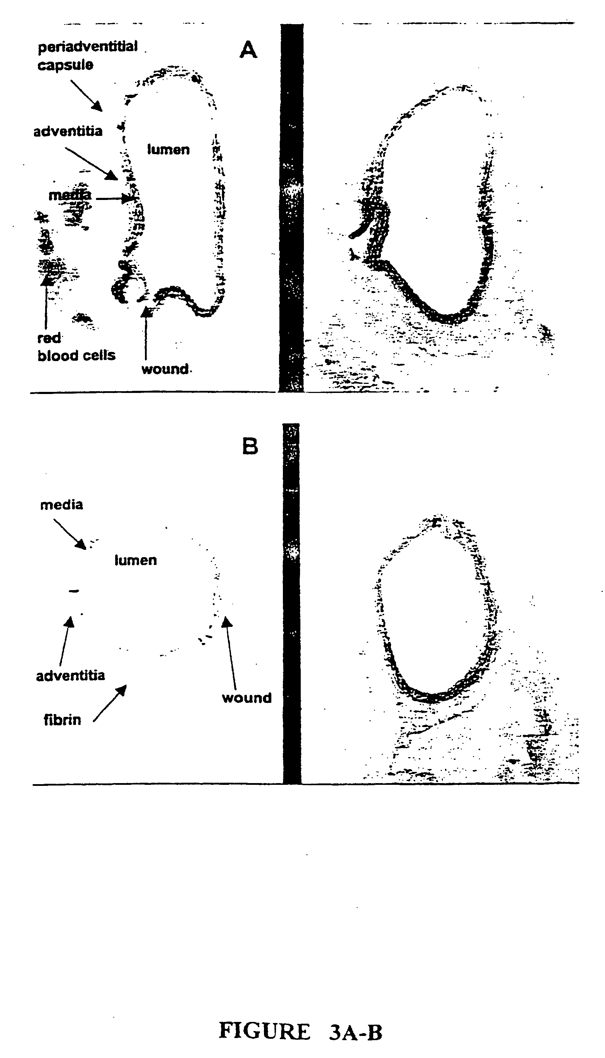

Figure 3 shows photomicrographs of aortic wounds in rats 14 days after arteriotomy and treatment: (A-left) wound treated with control EVA film devoid of paclitaxel compared to (A-right) untreated wound; (B-left) wound treated with 20% paclitaxel EVA compared to (B-right) untreated wound. Note the periadventitial capsule surrounding the aorta treated with control EVA film (A-left) as well as the red blood cells. Also note the acellular fibrin layer around the aorta treated with 20% paclitaxel EVA (B-left).

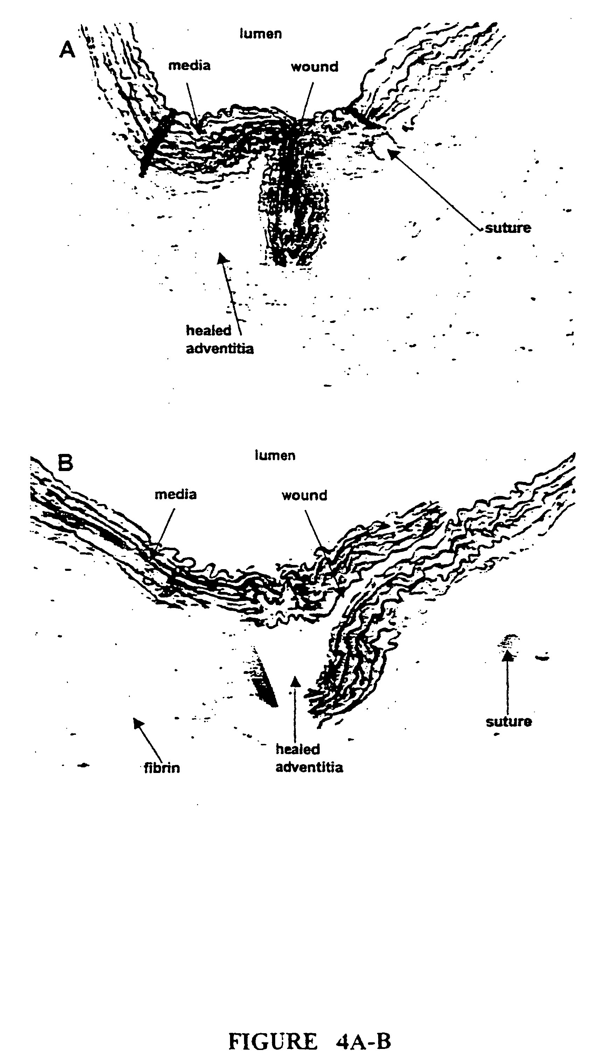

Figure 4 shows photomicrographs of aortic wounds 14 days after arteriotomy and treatment in (A) an untreated animal and (B) an animal treated with 20% paclitaxel EVA. The adventitia healed normally after treatment with paclitaxel (B). Note the fibrin layer around the treated aorta (B).

### DETAILED DESCRIPTION OF THE INVENTION

Prior to setting forth the invention, it may be helpful to an understanding thereof to set forth definitions of certain terms that will be used hereinafter.

"Body passageway" as used herein refers to any of number of passageways, tubes, pipes, tracts, canals, sinuses or conduits which have an inner lumen and allow the flow of materials within the body. Representative examples of body passageways include arteries and veins, lacrimal ducts, the trachea, bronchi, bronchiole, nasal passages (including the sinuses) and other airways, eustachian tubes, the external auditory canal, oral cavities, the esophagus, the stomach, the duodenum, the small intestine, the large intestine, biliary tracts, the ureter, the bladder, the urethra, the fallopian tubes, uterus, vagina and other passageways of the female reproductive tract, the vas deferens and other passageways of the male reproductive tract, and the ventricular system (cerebrospinal fluid) of the brain and the spinal cord.

"Body cavity" as used herein refers to any of number of hollow spaces within the body. Representative examples of cavities include, for example, the abdominal cavity, the buccal cavity, the peritoneal cavity, the pericardial cavity, the pelvic cavity, perivisceral cavity, pleural cavity, inguinal canal and uterine cavity.

"Therapeutic agent" as used herein refers to those agents which can mitigate, treat, cure or prevent a given disease or condition. Representative examples of therapeutic agents are discussed in more detail below, and include, for example, microtubule stabilizing agents, fibrosis inducers, angiogenic factors, growth factors and cytokines and other factors involved in the wound healing or fibrosis cascade.

As noted above, the present invention relates generally to compositions for improving the integrity of body passageways following surgery or injury, comprising the step of delivering to an eicternal portion of the body passageway (*i*.*e*., a nonluminal surface), a composition comprising a therapeutic agent, and within preferred embodiments, either a polymer alone or a compositions comprising a therapeutic agent (with or without a polymeric carrier). Briefly, delivery of a therapeutic agent to an external portion of a body passageway (*e*.*g*., quadrantically or circumferentially) avoids many of the disadvantages of traditional approaches. In addition, delivery of a therapeutic agent as described herein allows the administration of greater quantities of the therapeutic agent with less constraint upon the volume to be delivered.

As discussed in more detail below, a wide variety of therapeutic agents may be delivered to external portions of body passageways or cavities, either with or without a carrier (*e.g*., polymeric), in order to treat or prevent a condition associated with the body passageway or cavity. Each of these aspects is discussed in more detail below.

### THERAPEUTIC AGENTS

As noted above, the present disclosure provides methods and compositions which utilize a wide variety of therapeutic agents. Within one aspect of the disclosure the therapeutic agent is an microtubule stabilizing agent. Briefly, within the context of the present disclosure microtubule stabilizing agents should be understood to include any protein, peptide, chemical, or other molecule which acts to promote the stabilization of microtubules. A variety of methods may be readily utilized to determine the microtubule stabilizing activity of a given factor, including for example, tubulin assays. Briefly, fibroblasts are seeded in well plates housing coverslips. Following overnight incubation, the cells were treated with the compounds being evaluated for their effect on microtubules. After exposure, the cells are fixed, washed and stained with anti-tubulin antibody with a fluorescent marker. Signals are anlysed using a fluorescent microscope. In normal fibroblasts, microtubules can be observed as extensive fine, lace-like structural networks within the cytoplasm. Cells treated with microtubule stabilizing agents contain within them numerous microtubule organizing centers (MTOC).

In addition to the tubulin assay described above, a variety of other assays may also be utilized to determine the efficacy of microtubule stabilizing agents *in vitro*, including for example, assays described by Smith et al. (*Cancer Lett 79*(2):213-219, 1994) and Mooberry et al. (*Cancer Lett. 96*(2):261-266, 1995).

A wide variety of microtubule stabilizing agents may be readily utilized. Representative examples of such agents include taxanes (*e*.*g*., paclitaxel (discussed in more detail below) and docetaxel) (Schiff et al., *Nature* 277:665-667, 1979; Long and Fairchild, *Cancer Research* 54:4355-4361, 1994; Ringel and Horwitz, *J. Natl. Cancer Inst*. 83(4):288-291, 1991; Pazdur et al., *Cancer Treat. Rev*. 19(4):351-386, 1993), eleutherobin. (*e*.*g*., U.S. Patent No. 5,473,057), sarcodictyins (including sarcodictyin A), epothilone and analogues and derivatives thereof (Bollag et al., *Cancer. Research 55*:2325-2333, 1995), discodermolide (ter Haar et al., *Biochemistry 35*:243-250, 1996), deuterium oxide (D₂O) (James and Lefebvre, *Genetics* 130(2):305-314, 1992; Sollott et al., *J. Clin. Invert*. 95:1869-1876, 1995), hexylene glycol (2-methyl-2,4-pentanediol) (Oka et al., *Cell Struct. Funct*. 16(2):125-134, 1991), tubercidin (7-deazaadenosine) (Mooberry et al., *Cancer Lett*. 96(2):261-266, 1995), 2-amino-4-(3-pyridyl)-4H-naphtho(1,2-b)pyran-3-carbonitrile (Panda et al., *J. Biol. Chem*. 272(12):7681-7087, 1997; Wood et al., *Mol*. *Pharmacol*. 52(3):437-444, 1997), aluminum fluoride (Song et al., *J. Cell. Sci. Suppl.* 14:147-150, 1991), ethylene glycol bis-(succinimidylsuccinate) (Caplow and Shanks, J. *Biol. Chem*. 265(15):8935-8941, 1990), glycine ethyl ester (Mejillano et al., *Biochemistry* 31(13):3478-3483, 1992), LY195448 (Barlow & Cabral, *Cell Motil. Cytoskel*. 19:9-17, 1991), subtilisin (Saoudi et al., *J. Cell Sci*. 108:357-367, 1995), 1069C85 (Raynaud et al., *Cancer Chemother. Pharmacol*. 35:169-173, 1994), steganacin (Hamel, *Med. Res. Rev*. 16(2):207-231, 1996), combretastatins (Hamel, *Med*. *Res. Rev*. 16(2):207-231, 1996), curacins (Hamel, *Med. Res. Rev*. 16(2):207-231, 1996), estradiol (Aizu-Yokata et al., *Carcinogen*. 15(9):1875-1879, 1994), 2-methoxyestradiol (Hamel, *Med. Res. Rev*. 16(2):207-231, 1996), flavanols (Hamel, *Med. Res. Rev*. 16(2):207-231, 1996), rotenone (Hamel, *Med. Res. Rev*. 16(2):207-231, 1996), griseofulvin (Hamel, *Med. Res. Rev*. 16(2):207-231, 1996), vinca alkaloids, including vinblastine and vincristine (Ding et al., *J. Exp. Med*. 171(3):715-727, 1990; Dirk et al., *Neurochem. Res.* 15(11):1135-1139, 1990; Hamel, *Med. Res. Rev*. 16(2):207-231, 1996; Illinger et al., *Biol. Cell* 73(2-3):131-138, 1991; Wiemer et al., *J. Cell. Biol*. 136(1):71-80, 1997), maytansinoids and ansamitocins (Hamel, *Med. Res. Rev*. 16(2):207-231, 1996), rhizoxin (Hamel, *Med. Res. Rev.* 16(2):207-231, 1996), phomopsin A (Hamel, *Med. Res. Rev.* 16(2):207-231, 1996), ustiloxins (Hamel, *Med. Res. Rev.* 16(2):207-231, 1996), dolastatin 10 (Hamel, *Med. Res. Rev.* 16(2):207-231, 1996), dolastatin 15 (Hamel, *Med. Res. Rev.* 16(2):207-231, 1996), halichondrins and halistatins (Hamel, *Med. Res. Rev*. 16(2):207-231, 1996), spongistatins (Hamel, *Med. Res. Rev.* 16(2):207-231, 1996), cryptophycins (Hamel, *Med. Res. Rev.* 16(2):207-231, 1996), rhazinilam (Hamel, *Med. Res. Rev.* 16(2):207-231, 1996), betaine (Hashimoto et al., *Zool. Sci*. 1:195-204, 1984), taurine (Hashimoto et al., *Zool. Sci*. 1:195-204, 1984), isethionate (Hashimoto et al., *Zool. Sci.* 1:195-204, 1984), HO-221 (Ando et al., *Cancer Chemother. Pharmacol.* 37:63-69, 1995), adociasulfate-2 (Sakowicz, et al., *Science* 280:292-295, 1998), estramustine (Panda et al., *Proc. Natl. Acad. Sci. USA* 94:10560-10564, 1997), monoclonal anti-idiotypic antibodies (Leu et al., *Proc. Natl. Acad. Sci. USA* 91(22):10690-10694, 1994), microtubule assembly promoting protein (taxol-like protein, TALP) (Hwang et al., *Biochem. Biophys. Res. Commun.* 208(3):1174-1180, 1995), cell swelling induced by hypotonic (190 mosmol/L) conditions, insulin (100 nmol/L) or glutamine (10 mmol/L) (Haussinger et al., *Biochem. Cell. Biol.* 72(1-2):12-19, 1994), dynein binding (Ohba et al., *Biochim. Biophys. Acta* 1158(3):323-332, 1993), gibberelin (Mita and Shibaoka, *Protoplasma* 119(1/2):100-109, 1984), XCHO1 (kinesin-like protein) (Yonetani et al., *Mol. Biol. Cell* 7(suppl):211A, 1996), lysophosphatidic acid (Cook et al., *Mol. Biol. Cell* 6(suppl):260A, 1995), lithium ion (Bhattacharyya and Wolff, *Biochem. Biophys. Res. Commun.* 73(2):383-390, 1976), plant cell wall components *(e.g.,* poly-L-lysine and extensin) (Akashi et al., *Planta* 182(3):363-369, 1990), glycerol buffers (Schilstra et al., *Biochem. J*. 277(Pt. 3):839-847, 1991; Farrell and Keates, *Biochem. Cell. Biol*. 68(11):1256-1261, 1990; Lopez et al., *J. Cell. Biochem.* 43(3):281-291, 1990), Triton X-100 microtubule stabilizing buffer (Brown et al., *J. Cell Sci*. 104(Pt. 2):339-352, 1993; Safiejko-Mroczka and Bell, *J. Histochem. Cytochem.* 44(6):641-656, 1996), microtubule associated proteins *(e.g.,* MAP2, MAP4, tau, big tau, ensconsin, elongation factor-1-alpha (EF-1α) and E-MAP-115) (Burgess et al., *Cell Motil. Cytoskeleton* 20(4):289-300, 1991; Saoudi et al., *J. Cell. Sci.* 108(Pt. 1):357-367, 1995; Bulinski and Bossler, *J*. *Cell. Sci.* 107(Pt. 10):2839-2849, 1994; Ookata et al., *J. Cell Biol.* 128(5):849-862, 1995; Boyne et al., *J. Comp. Neurol.* 358(2):279-293, 1995; Ferreira and Caceres, *J. Neurosci.* 11(2):392-400, 1991; Thurston et al., *Chromosoma* 105(1):20-30, 1996; Wang et al., *Brain Res. Mol. Brain Res.* 38(2):200-208, 1996; Moore and Cyr, *Mol. Biol. Cell* 7(suppl):221-A, 1996; Masson and Kreis, *J*. *Cell Biol.* 123(2), 357-371, 1993), cellular entities (*e*.*g*., histone H1, myelin basic protein and kinetochores) (Saoudi et al., *J. Cell. Sci*. 108(Pt. 1):357-367, 1995; Simerly et al., *J. Cell Biol*. 111(4):1491-1504, 1990), endogenous microtubular structures (*e*.*g*., axonemal structures, plugs and GTP caps) (Dye et al., *Cell Motil. Cytoskeleton* 21(3):171-186, 1992; Azhar and Murphy, *Cell Motil. Cytoskeleton* 15(3):156-161, 1990; Walker et al., *J. Cell Biol*. 114(1):73-81, 1991; Drechsel and Kirschner, *Curr. Biol*. 4(12):1053-1061, 1994), stable tubule only polypeptide (*e*.*g*., STOP145 and STOP220) (Pirollet et al., *Biochim. Biophys. Acta* 1160(1):113-119, 1992; Pirollet et al., *Biochemistry* 31(37):8849-8855, 1992; Bosc et al., *Proc. Natl. Acad. Sci. USA* 93(5):2125-2130, 1996; Margolis et al., *EMBO J.* 9(12):4095-4102, 1990) and tension from mitotic forces (Nicklas and Ward, *J. Cell Biol*. 126(5):1241-1253, 1994), as well as any analogues and derivatives of any of the above.

Within one preferred embodiment , the therapeutic agent is paclitaxel, a compound which disrupts microtubule formation by binding to tubulin to form abnormal mitotic spindles. Briefly, paclitaxel is a highly derivatized diterpenoid (Wani .et al., *J. Am. Chem. Soc. 93*:2325, 1971) which has been obtained from the harvested and dried bark of *Taxus brevifolia* (Pacific Yew) and *Taxomyces Andreanae* and *Endophytic Fungus* of the Pacific Yew (Stierle et al., *Science 60*:214-216, 1993). "Paclitaxel" (which should be understood herein to include prodrugs, analogues and derivatives such as, for example, TAXOL®, TAXOTERE®, Docetaxel, 10-desacetyl analogues of paclitaxel and 3'N-desbenzoyl-3'N-t-butoxy carbonyl analogues of paclitaxel) may be readily prepared utilizing techniques known to those skilled in the art *(see e.g.,* Schiff et al., *Nature 277*:665-667, 1979; Long and Fairchild, *Cancer Research 54*:4355-4361, 1994; Ringel and Horwitz, *J. Natl. Cancer Inst. 83*(4):288-291, 1991; Pazdur et al., *Cancer Treat. Rev. 19*(4):351-386, 1993; WO 94/07882; WO 94/07881; WO 94/07880; WO 94/07876; WO 93/23555; WO 93/10076; WO94/00156; WO 93/24476; EP 590267; WO 94/20089; U.S. Patent Nos. 5,294,637; 5,283,253; 5,279,949; 5,274,137; 5,202,448; 5,200,534; 5,229,529; 5,254,580; 5,412,092; 5,395,850; 5,380,751; 5,350,866; 4,857,653; 5,272,171; 5,411,984; 5,248,796; 5,248,796; 5,422,364; 5,300,638; 5,294,637; 5,362,831; 5,440,056; 4,814,470; 5,278,324; 5,352,805; 5,411,984; 5,059,699; 4,942,184; *Tetrahedron Letters 35*(52):9709-9712, 1994; *J. Med. Chem. 35*:4230-4237, 1992; *J. Med. Chem. 34*:992-998, 1991; *J. Natural Prod. 57*(10):1404-1410, 1994; *J. Natural Prod. 57*(11):1580-1583, 1994; *J. Am. Chem. Soc. 110*:6558-6560, 1988), or obtained from a variety of commercial sources, including for example, Sigma Chemical Co., St. Louis, Missouri (T7402 - from *Taxus brevifolia*).

Representative examples of such paclitaxel derivatives or analogues include 7-deoxy-docetaxol, 7,8-cyclopropataxanes, N-substituted 2-azetidones, 6,7-epoxy paclitaxels, 6,7-modified paclitaxels, 10-desacetoxytaxol, 10-deacetyltaxol (from 10-deacetylbaccatin III), phosphonooxy and carbonate derivatives of taxol, taxol 2',7-di(sodium 1,2-benzenedicarboxylate, 10-desacetoxy-11,12-dihydrotaxol-10,12(18)-diene derivatives, 10-desacetoxytaxol, Protaxol (2'-and/or 7-O-ester derivatives ), (2'- and/or 7-O-carbonate derivatives), asymmetric synthesis of taxol side chain, fluoro taxols, 9-deoxotaxane, (13-acetyl-9-deoxobaccatine III, 9-deoxotaxol, 7-deoxy-9-deoxotaxol, 10-desacetoxy-7-deoxy-9-deoxotaxol, Derivatives containing hydrogen or acetyl group and a hydroxy and tert-butoxycarbonylamino, sulfonated 2'-acryloyltaxol and sulfonated 2'-O-acyl acid taxol derivatives, succinyltaxol, 2'-γ-aminobutyryltaxol formate, 2'-acetyl taxol, 7-acetyl taxol, 7-glycine carbamate taxol, 2'-OH-7-PEG(5000) carbamate taxol, 2'-benzoyl and 2',7-dibenzoyl taxol derivatives, other prodrugs (2'-acetyltaxol; 2',7-diacetyltaxol; 2'succinyltaxol; 2'-(beta-alanyl)-taxol); 2'gamma-aminobutyryltaxol formate; ethylene glycol derivatives of 2'-succinyltaxol; 2'-glutaryltaxol; 2'-(N,N-dimethylglycyl) taxol; 2'-(2-(N,N-dimethylamino)propionyl)taxol; 2'orthocarboxybenzoyl taxol; 2'aliphatic carboxylic acid derivatives of taxol, Prodrugs {2'(N,N-diethylaminopropionyl)taxol, 2'(N,N-dimethylglycyl)taxol, 7(N,N-dimethylglycyl)taxol, 2',7-di-(N,N-dimethylglycyl)taxol, 7(N,N-diethylaminopropionyl)taxol, 2',7-di(N,N-diethylaminopropionyl)taxol, 2'-(L-glycyl)taxol, 7-(L-glycyl)taxol, 2',7-di(L-glycyl)taxol, 2'-(L-alanyl)taxol, 7-(L-alanyl)taxol, 2',7-di(L-alanyl)taxol, 2'-(L-leucyl)taxol, 7-(L-leucyl)taxol, 2',7-di(L-leucyl)taxol, 2'-(L-isoleucyl)taxol, 7-(L-isoleucyl)taxol, 2',7-di(L-isoleucyl)taxol, 2'-(L-valyl)taxol, 7-(L-valyl)taxol, 2'7-di(L-valyl)taxol, 2'-(L-phenylalanyl)taxol, 7-(L-phenylalanyl)taxol, 2',7-di(L-phenylalanyl)taxol, 2'-(L-prolyl)taxol, 7-(L-prolyl)taxol, 2',7-di(L-prolyl)taxol, 2'-(L-lysyl)taxol, 7-(L-lysyl)taxol, 2',7-di(L-lysyl)taxol, 2'-(L-glutamyl)taxol, 7-(L-glutamyl)taxol, 2',7-di(L-glutamyl)taxol, 2'-(L-arginyl)taxol, 7-(L-arginyl)taxol, 2',7-di(L-arginyl)taxol}, Taxol analogs with modified phenylisoserine side chains, taxotere, (N-debenzoyl-N-tert-(butoxycaronyl)-10-deacetyltaxol, and taxanes (*e*.*g*., baccatin III, cephalomannine, 10-deacetylbaccatin III, brevifoliol, yunantaxusin and taxusin).

A wide variety of agents that induce fibrosis such as camptothecin, or an analogue or derivative thereof may also be utilized within the context of the present invention. Representative examples of such agents include irritants, such as talcum powder (Chlapik and Gogora, *Rozhl*. *Chir.* 69 (5):322-326, 1990), metallic beryllium and silica (Nemery, *Eur. Resp*. *J*. 3(2):202-219, 1990); components of extracellular matrix, such as fibronectin (Driscoll et al., *J*. *Toxicol*. *Environ. Health* 46 (2):155-169, 1995); polymers *[e.g.,* poly(lysine) and poly(ethylene vinyl acetate)]; inflammatory cytokines, such as transforming growth factor-β (TGF-β) (Fausto et al., *Ciba Found. Symp.* 157:165-174, 1991), platelet-derived growth factor (PDGF) (Tang et al., *American Journal of Pathology* 148 (4):1169-1180, 1996), vascular endothelial growth factor/vascular permeability factor (VEGF/VPF) (Grone et al., *Journal of Pathology* 177 (3):259-267, 1995), basic fibroblast growth factor (bFGF) (Inoue et al., *American Journal of Pathology* 149 (6):2037-2054, 1996), tumor necrosis factor α (TNF α) (Thrall et al., *American Journal of Pathology* 151 (5):1303-1310, 1997), tumor necrosis factor β (TNF β) (Franko et al., *Radiation Research* 147 (2):245-256, 1997, nerve growth factor (NGF) (Liu et al., *Acta Neuropathol. (Berl.)* 88 (2):143-150, 1994), granulocyte macrophage colony stimulating factor (GM-CSF) (Xing et al., *American Journal of Pathology* 150 (1):59-66, 1997; Xing et al., *Journal of Clinical Investigation* 97 (4):1102-1110, 1996), epithelial growth factor (EGF) (Magro et al., *Journal of Pathology* 181 (2):213-217, 1997), insulin-like growth factor-1 (IGF-1) (Laursen et al., *Arch. Dis. Child*. 72 (6):494-497, 1995. Homma et al., *Am. J*. *Respir. Crit. Care Med.* 152 (6) Pt 1:2084-2089, 1995), interleukin 1 (IL-1) (Smith et al., *Am. J*. *Respir. Crit. Care Med.* 151 (6):1965-1973, 1995), IL-8 (Lonnemann et al., *Kidney Int*. 47 (3):845-854, 1995), IL-6 (Nixon et al., *Am. J*. *Respir. Crit. Care Med.* 157 (6) Pt 1, 1764-1769, 1998), growth hormone (GH) (Culler and Meacham, *Neuroendocrinology* 58 (4):473-477, 1993); and inflammatory microcrystals (*e*.*g*., crystalline minerals, such as crystalline silicates). Other representative examples include monocyte chemotactic protein-1 (MCP-1) (Lloyd et al., *J*. *Leukoc*. *Biol*. *62* (5):676-680, 1997); fibroblast stimulating factor-1 (FSF-1) (Greenwel et al., *Infect. Immun.* 61 (9):3985-3987, 1993); histamine (Jacquot et al., *FEBS Lett.* 386 (2-3):123-127, 1996; Broide et al., *J*. *Immunol*. 145 (6):1838-1844, 1990); heparin (Piguet et al., *Int*. *J*. *Exp. Pathol*. 77 (4):155-161, 1996); fibrin/fibrinogen (Imokawa et al., *Am. J. Respir. Crit. Care Med.* 156 (2) Pt 1:631-636, 1997; Neubauer et al., *Gastroenterology* 108 (4):1124-1135, 1995), endothelin-1 (Mutsaers et al., *Am*. *J*. *Respir. Cell Mol. Biol.* 18 (5):611-619, 1998); fibrosin (Prakash et al., *Proc. Natl*. *Acad. Sci. USA* 92(6):2154-2158, 1995); angiotensin II (Campbell et al., *J*. *Mol*. *Cell Cardiol*. 27 (8):1545-1560, 1995); iron overload (Arthur, *J. Gastroenterol. Hepatol.* 11 (12):1124-1129, 1996); opsonized zymozan (Jiang et al., *J*. *Immunol*. *Methods* 152 (2):201-207, 1992); condress (Beghe et al., *Int*. *J*. *Tiss*. *React*. 14 Suppl.:11-19, 1992); bromocriptine (Hillerdal et al., *Eur. Resp. J*. 10 (12):2711-2715, 1997); methysergide (Muller et al., *Dtsch*. *Med. Wochenschr.* 116 (38):1433-1436, 1991; Bucci and Manoharan, *Mayo. Clin*. *Proc.* 72 (12):1148-1150, 1997); methotrexate (van der Veen et al., *J*. *Rheumatol.* 22 (9):1766-1768, 1995); N-carboxybutyl chitosan (Biagini et al., *Biomaterials* 12 (3):287-291, 1991); carbon tetrachloride (Paakko et al., *Arch. Toxicol.* 70 (9):540-552, 1996); thioacetamide (Muller et al., *J. Hepatol.* 25 (4):547-553, 1996); quartz dust (Hurych et al., *Toxicol. Lett.* 11 (1-3):305-311, 1996); carbon tetracholoride (Odenthal et al., *Gastroenterology* 102 (4) Part 2:A863, 1992); bleomycin (Santana et al., *Am. Rev. Respir. Dis.* 145 (4) Part 2:A442, 1992); azathioprine (Mion et al., *Gut* 32 (6):715-717, 1991); ethionine (Ikeno et al., *Gastroenteology* 100 (5) Part 2:A277, 1991); paraquat (Hudson et al., *Thorax* 46 (3):201-204, 1991); thorotrast (De Vuyst et al., *Thorax 45* (11):899-901, 1990); iron dextran complex (Carthew et al., *Hepatology* 13 (3):534-539, 1991); cadmium chloride (Damiano et al., *Am. J. Pathol*. 137 (4):883-894, 1990); chlorhexidine (Farrell et al., *FASEB* 4 (3):A670, 1990); amiodarone (Lau et al., *J. Hong Kong Med. Assoc.* 41 (2):181-184, 1989); tetracycline (Baumann et al., *Am. Rev. Respir. Dis.* 139 (4) Part 2:A359, 1989); hapten (Boucher et al., *Am. Rev. Respir. Dis.* 135 (4) Part 2:A140, 1987); melphalan (Mufti et al., *Acta Hematol. (Basel)* 69 (2):140-141, 1983); vinyl chloride (Okudaira, *J. UOEH 4* (Suppl.):135-146, 1982); saponin (Wang & Tobin, *Br. J. Haematol.* 51 (2):277-284, 1982); isoproterenol (Boyd et al., *Teratology* 24 (2):10A, 1981); cyclophosphamide (Spector & Zimbler, *Proc. Am. Assoc. Cancer Res. Clin. Oncol*. 22:362, 1981); carmustine (Klein & Paddison, *Arch. Neurol.* 38 (6):393-393, 1981); N-nitroso-N-methyl urethane (Cantor et al., *Proc. Soc. Exp. Biol. Med.* 164 (1):1-8, 1980); pentacozine (Rousseau et al., *Arch. Neurol*. 36 (11):723-724, 1979); thiouracil (Lunkenheimer et al., *Pathol. Res. Pract.* 163 (1):47-56, 1978); lithium (Hestbach et al., *Acta Pathol. Microbiol. Scand. Sect. A Pathol.* 86 (2):195-198, 1978); dilantin (Hassell et al., *J. Dent. Res.* 56 (Special Issue A):A145, 1977); methysergide (Paccalin et al., *Therapie (Paris)* 31 (2):231-239, 1976); methyl-4-dimethlyamino azo benzene (Terao & Nakano, *GANN* 65 (3):249-260, 1974); poly chlorinated biphenyls aroclor (Kimbrough & Linder, *J. Natl. Cancer Inst.* 53 (2):547-552, 1974); butylated hydroxytoluene (BHT) (Okada et al., *Ketsugo Soshiki* 17 (3):167-179, 1985); cyclochlorotine (Terao & Ito, *Maikotokishin (Tokyo)* 17:59-61, 1983); β-blocker (Proulx & Schneiweiss, *Drug Intell. Clin. Pharm.* 19:359-360, 1985); nitrofurantoin (Robinson, *Medical Journal of Australia (Australia)* 1:72-76, 1983); timolol (Rimmer et al., *Lancet (England)* 1:300, 1983); trisodium citrate and acid-citrate dextrose (Mitsuhashi et al., *Exp. Mol. Pathol.* 42 (2):261-270, 1985); peplomycin (Ekimoto et al., *J. Antibiot. (Tokyo)* 38 (1):94-98, 1985); amiodarone and desethylamiodarone (Daniels et al., *Toxicol. Appl. Pharmacol*. 100 (2):350-359, 1989); chlorambucil (Carr., *Va. Med*. 113 (11):677-680, 1986); dimethlynitrosamine (Ala-Kokko et al., *Biochem. J*. 244 (1):75-79, 1987); diquat (Manabe & Ogata., *Arch. Toxicol.* 60 (6):427-431, 1987); meperidine (Yamanaka & Parsa, *Plast. Reconstr. Surg.* 75 (4):582-583, 1985); vinyl chloride (Jones & Smith, *Br. J. Ind. Med*. 39 (3):306-307, 1982); butylated hydroxytoluene and oxygen (Haschek et al., *Am. J. Pathol*. 105 (3):333-335, 1981); carmustine (Patten et al., *JAMA* 244 (7):687-688, 1980); dibutyltin dichloride (Yermakoff et al., *Toxicol. Appl. Pharmacol*. 49 (1):31-40, 1979); allylamine (Lalich & Paik, *Exp. Mol. Pathol*. 21 (1):29-39,1974); catecholamines (Gvozdjak et al., *Arch. Mal. Coeur Vaiss*. 64 (2):269-277, 1971) and minerals (Glass et al., *Occup. Environ. Med.* 52 (7):433-440, 1995; Craighead et al., *Hum. Pathol*. 23 (10):1098-1105, 1992).

A wide variety of angiogenic factors may be readily utilized within the context of the present disclosure. Representative examples of direct angiogenesis stimulating factors include growth/differentiation factor (GDF)-5 (Yamashita, H. et al. *Exp Cell Res*, *235*(1):218-226, 1997); hydrogen peroxide, doxorubicin (Monte, M., et al. *Eur. J. Cancer* 33(4):676-682, 1997); IL-8, bFGF, TNFα, IL-1 (Norrby, K. *Microvasc. Res. 54*(1):58-64, 1997); placental-derived growth factor (PIGF) (Ziche, M. et al. *Lab. Invest. 76*(4):517-31, 1997); VEGF/VPF (Brown, L.F., et al. *EXS 79*:233-69, 1997; Samaniego, F., et al. *Am. J. Pathol. 152*(6):1433-43, 1998); extracellular matrix-degrading enzymes, MMP-2 and MMP-9 (Ribatti, D., et al. *Int. J. Cancer 77*(3):449-54, 1998); aFGF, heparin (Rosengart, T.K., et al. *J. Vasc. Surg. 26*(2):302-12, 1997); estrogens (Banerjee, S.K., et al. *Carcinogenesis 18*(6):1155-61, 1997); lidocaine, bFGF (Jejurikar, S.S., et al. *J. Surg. Res. 67*(2):137-46, 1997); degradation products of hyaluronan of 3 to 10 disaccharides (o-HA) (Slevin, M., et al. *Lab. Invest. 78*(8):987-1003, 1998); urokinase (uPA) (Rabbani, S.A. *In Vivo 12*(1):135-42, 1997); elastin degradation products (Nackman, G.B., et al. *Surgery 122*(1):39-44, 1997); advanced glycation end products (AGE) (Yamagishi, Si., et al. *J. Biol. Chem. 272*(13):8723-30, 1997); angiopoietin-1 (Koblizek, T.I., et al. *Curr. Biol. 8*(9):529-32, 1998); FGF 2/FGF 4 (Bagheri, Y.R., et al. *Br. J. Cancer 78*(1):111-8, 1998); IGF-II (Bae, M.H., et al. *Cancer Lett. 128*(1):41-6, 1998); pleiotrophin (PTN) (Yeh, H.J., et al. *J. Neurosci. 18*(10):3699-707, 1998); FGF-2, FGF-1 (Jouanneau, J., et al. *Oncogene 14*(6):671-6, 1997); chemokines, MGSA/GRO alpha, -beta and -gamma (Owen, J.D., et al. *Int. J. Cancer 73*(1):94-103, 1997); heparin and cholesterol (Tyagi, S.C., et al. *Mol. Cell. Cardiol. 29*(1):391-404, 1997); gamma-IFN (Fiorelli, V., et al. *Blood 91*(3):956-67, 1998); IL-2, IL-6, IL-8 (Rizk, B., et al. *Hum. Reprod. Update 3*(3):255-66, 1997); E-type prostaglandins, ceruloplasmin (Ziche, M., et al. *J. Natl. Cancer Inst. 69*(2):475-82, 1982); bovine endothelium stimulating factor (McAuslan, B.R., et al. *Microvasc. Res. 26*(3):323-38, 1983); CXC chemokines (except IP-10) (Stricter, R.M., et al. *Shock 4*(3):155-60, 1995); angiogenins (Reisdorf, C., et al. *Eur. J. Biochem. 224*(3):811-22, 1994); fibrin, zymosan activated serum, an N-formylmethionine tripeptide, PDGF (Dvorak, H.K., et al. *Lab. Invest. 57*(6):673-86, 1987); PGE2 (Form, D.M., Auerbach, *R. Proc. Soc. Exp. Biol. Med. 172*(2):214-18, 1983); int-2 oncogene (Costa, M., et al., *Cancer Res. 54*(1):9-11, 1994); tumor angiogenesis factor (TAF) (Byrne, H.M., Chaplain, M.A. *Bull. Math. Biol. 57*(3):461-86, 1995); phorbol esters (Morris, P.B., et al. *Am. J. Physiol. 254*(2) Pt.1:C318-22, 1988); bovine brain derived class 1 heparin-binding growth factor (Lobb, R.R., et al. *Biochemistry 24*(19):4969-73, 1985); bacterial endotoxin lipopolysaccharide (LPS) and thrombospondin (BenEzra, D., et al. *Opthalmol. Vis. Sci. 34*(13):3601-8, 1993); platelet-activating factor (Camussi, G., et al. *J. Immunol. 154*(12):6492-501, 1995); SPARC peptides (Iruela Arispe, M.L., et al. *Mol. Biol. Cell 6*(3):327-43, 1995); TGF-beta 1 (Pepper, M.S., et al. *J. Cell. Biol. 111*(2):743-55, 1990); urokinase plasminogen activator (uPA) (Hildenbrand, R., et al. *Pathol. Res, Pract. 191*(5):403-9, 1995); hepatocyte growth factor (HGF) (Silvagno, F., et al. *Arterioscler. Thromb. Vasc. Biol. 15*(11):1857-65, 1995); thymidine phosphorylase (dThdPase) (Takebayashi, Y., et al. *Cancer Lett.* 95(1-2):57-62, 1995); epidermal growth factor (EGF) (Reilly, W., McAustan, B.R. *Adv. Exp. Med. Biol.* 242:221-7, 1988); crocidolite asbestos fibers, chrysotile asbestos, fiberglass (Branchaud, R.M., et al. *FASEB J*. *3*(6):1747-52, 1989); angiotropin (Hockel, M., et al. *J. Clin. Invest. 82*(3):1075-90, 1988); spermine, spermidine (Takigawa, M., et al. *Biochem. Biophys. Res. Commun. 171*(3):1264-71, 1990); degradative enzymes, E-prostaglandins, fibronectin, metal cations (Obrenovitch, A., Monsigny, M. *Pathol. Biol. (Paris) 34*(3):189-201, 1986); endothelial cell-stimulating angiogenic factor (ESAF) (Taylor, C.M., et al. *Invest. Ophthalmol. Vis. Sci. 30*(10):2174-8, 1989); chondrosarcoma-derived growth factor (ChDGF) (Shing, Y., et al. *J. Cell. Biochem. 29*(4):275-87, 1985); PDGF AA, AB, BB (Oikawa, T., et al. *Biol. Pharm. Bull. 17*(12):1686-8, 1994); angiogenin binding protein (AngBP) (Hu, G.F., et al. *Proc. Natl. Acad. Sci. USA* 88(6):2227-31, 1991); scatter factor (Grant, D.S., et al. *Proc. Natl. Acad. Sci. USA 90*(5):1937-41, 1993); nicotinamide (Kill, F.C. Jr., et al. *Science 236*(4803):843-5, 1987); phorbol myristate acetate (PMA) (Montesano, R., Orci, L. *Cell 42*(2):469-77, 1985); angiogenic factor (AF) (Arnold, F., et al. *Int. J. Microcirc. Clin. Exp. 5*(4):381-6, 1987); erucamide (13-docosenamide) (Wakamatusu, K., et al. *Biochem. Biophys. Res. Commun. 168*(2):423-9, 1990); class I heparin-binding growth factor (HBGF-I) (Winkles, J.A., et al. *Proc. Natl. Acad. Sci. USA 84*(20):7124-8, 1987); low molecular weight fibrin degradation products (Thompson, W.D., et al. *J. Pathol. 145*(1):27-37, 1985); vanadate (Montesano, R., et al. *J. Cell Physiol. 134*(3):460-6, 1988); 7,12-dimethylbenz[a]anthracene (DMBA) (Polverini, P.J., Solt, D.B. *Carcinogenesis 9*(1):117-22, 1988); retinoic acid (Kligman, L.H. *J. Am. Acad*. *Dermatol. 21*(3) Pt.2:623-31, 1989); PDWHF which includes PDGF, PDAF, PF4 (Hiraizumi, Y., et al. *Spinal Cord 34*(7):394-402, 1996); proliferin (Volpert, O., et al. *Endocrinology 137*(9):3871-6, 1996); elastin degradation products (Nackman, G.B., et al. *Ann. NY Acad. Sci. 800*:260-2, 1996). Representative agents that indirectly stimulate angiogenesis include TNF, IL-1, IFN-gamma (Samaniego, F., et al. *Am. J. Pathol. 152*(6):1433-43, 1998); cDNA coding for angiogenic factors (Melillo, G., et al. *Cardiovasc. Res. 35*(3):480-9, 1997); alphalbetal and alpha2beta1 integrins (Senger, D.R., et al. *Proc. Natl. Acad. Sci. USA 94*(25):13612-7, 1997); prostaglandins, adenosine, TGF-alpha, bFGF, TGF-beta, TNF-alpha, KGF, PDGF (Brown, L.F., et al. *EXS 79*:233-69, 1997); G-protein-coupled receptor of Kaposi's sarcoma-associated herpesvirus, JNK/SAPK, p38MAPK (Bais, C., et al. *Nature 391*(6662):86-9, 1998); estrogens (Banerjee, S.K., et al. *Carcinogenesis 18*(6):1155-61, 1997); IL-1 alpha, IL-1 beta, TNF-alpha, TNF-beta (Ferrer, F.A., et al. *J. Urol. 157*(6):2329-33, 1997); matrix metalloproteinases MT1-MMP, MMP-2 (Haas, T.L., et al. *J. Biol. Chem. 273*(6):3604-10, 1998); platelet-derived endothelial cell growth factor (PD-ECGF) (Nakayama, Y., et al. *Surg. Neurol. 49*(2):181-8, 1998); human ornithine decarboxylase (ODC) (Auvinen, M., et al. *Cancer Res. 57*(14):3016-25, 1997); Hox D3 homeobox gene (Boudreau, N., et al. *J. Cell. Biochem. 139*(1):257-64, 1997); heme oxygenase (HO-1) (Deramaudt, B.M., et al. *J. Cell. Biochem. 68*(1):121-7, 1998); FGF-4 (Deroanne, C.F., et al. *Cancer Res. 57*(24):5590-7, 1997); hypoxia and interleukin 1 beta (IL-1beta) (Jackson, J.R., et al. *J. Rheumatol. 24*(7):1253-9, 1997); NF-kappaB (Bhat Nakshatri, P., et al. *Proc. Natl. Acad. Sci. USA 95*(12):6971-6, 1998); alphavbeta3 integrin (Scatena, M., et al. *J. Cell. Biol. 141*(4):1083-93, 1998); tissue factor (TF) (Poulson, L.K., et al. *J. Biol. Chem. 273*(11):6228-32, 1998); acetyl-NT (8-13) analogue, TJN-950 (Ushiro, S., et al. *FEBS Lett. 18*(3):341-5, 1997); hepatocyte growth factor (HGF), epidermal growth factor (EGF) (Takahashi, M., et al. *FEBS Lett. 418*(1-2):115-118, 1997); COX-2 (Katori, M., et al. *Nippon Yakurigaku Zasshi 109*(6):247-58, 1997); c-ets1 transcription factor (Calmels, T.P., et al. *Biol. Cell 84* (1-2):53-61, 1995); perlecan (Aviezer, D., et al. *Cell 79*(6):1005-13, 1994); adenosine, inosine, hypoxanthine, nicotinamide, lactic acid, phorbol esters, prostaglandin E2, copper (Terrell, G.E., Swain, J.L. *Matrix 11*(2):108-14, 1991); PDGF (Sato, N., et al. *Am. J. Pathol. 142*(4):1119-30, 1993); phorbol 12-myristate 13-acetate (Winkles, J.A. et al. *Cancer Res. 52*(4):1040-3, 1992); leukotrienes derived from arachidonic acid (Modat,G., et al. *Prostaglandins 33*(4):531-8, 1987); urokinase-type plasminogen activator (uPA), matalloproteinases, collagenases, gelatinases, stromelysin (Menashi, S., et al. *Baillieres Clin. Haematol. 6*(3):559-76, 1993); dobutamine, alinidine (Brown, M.D., Hudlicka, O. *EXS 61*:389-94, 1993); and omentopexy (Mayer, E., et al. *J. Thorac. Cardiovasc. Surg. 104*(1):180-8, 1992).

A wide variety of cytokines and other factors involved in the wound healing or fibrosis cascade may be readily utilized within the context of the present disclosure. Representative examples include TGF-beta (Bilgihan, K., et al. *Ophthalmologica 211*(6):380-3, 1997); FGF (Gospodarowicz, D., et al. *Prog. Clin. Biol. Res. 9*:1-19, 1976); angiotropin (Hockel, M., et al. *J. Clin. Invest. 82*(3):1075-90, 1988); bFGF (Knighton, D.R., et al. *J. Trauma 30*(12)Suppl.:S134-44, 1990); laminin SIKVAV peptide (Corcoran, M.L., et al. *J. Biol. Chem. 270*(18):10365-8, 1995); angiogenic factor (AF) (Arnold, F., et al. *Microcirc. Clin. Exp. 5*(4):381-6, 1987); PDGF, EGF, TGF-alpha, TNF, interferons (Nagy, J.A., et al. *Biochim. Biophys. Acta. 948*(3):305-26, 1989); lymphilized type I collagen (Mian, E., et al. *Int. J. Tissue React. 13*(5):257-69, 1991); mast cell activator - compound 48/80 (Clinton, M., et al. *Int. J. Microcirc. Clin. Exp. 7*(4):315-26, 1988); ascorbate (Appling, W.D., et al. *FEBS Lett*. *250*(2):541-4, 1989); arginine, vitamins A, B, C (Meyer, N.A., et al. *New Horiz. 2*(2):202-14, 1994); heparin binding growth factors (HBGFs), chemically substituted dextrans (Meddahi, A., et al. *Pathol. Res. Pract. 190*(9-10):923-8, 1994); recombinant human platelet-derived growth factor BB (rP-DGF-BB) (Pierce, G.F., et al. *Am. J. Pathol. 145*(6):1399-140, 1994); insulin (Weringer, E.J., et al. *Diabetes 30*(5):407-410, 1981); Cu Zn-superoxide dismutase (Nishiguchi, K., et al. *Pharmaceutical Research (USA) 11*:1244-49, 1994); platelet-derived wound healing factors (procuren) (Gillam, A.J., et al. *Annals of Pharmacotherapy 27*:1201-3, 1993); polypeptide growth factors (Glick, A.B., et al. *Cosmetics & Toiletries (USA) 109*:55-60, 1994); keratinocyte growth factor (KGF) (Egger, B., et al. *Am. J. Surg. 176*(1):18-24, 1998); nerve growth factor (NGF) (Matsuda, H., et al. *J. Exp. Med. 187*(3):297-306, 1998); macrophage colony-stimulating factor (M-CSF) (Wu, L., et al. *J. Surg. Res. 72*(2):162-9, 1997); hepatocyte growth factor (Kinoshita, Y., et al. *Digestion 58*(3):225-31, 1997); macrophage migration inhibitory factor (MIF) (Matsuda, A., et al. *Invest. Ophthalmol. Vis. Sci. 38*(8):1555-62, 1997); VEGF (Takahashi, M., et al. *Biochem. Biophys. Res. Commun. 19234*(2):493-8, 1997); TGF-beta 1 and 2 isoforms (Ashcroft, G.S., et al. *J. Anat. 190*(Pt 3):351-65, 1997); endothelial cell growth factor (ECGF) (Ko, C.Y., et al. *J. Cont. Rel. 44*(2-3):209-14, 1997); IL-1B (Press release, *Cistron Biotechnology*, 1998); GM-CSF (El Saghir, N.S., et al. *J. Infect. 35*(2):179-82, 1997); factor XIIIA _{.} (Chamouard, P., et al. *J. Gastroenterol. 93*(4):610-4, 1998); polypeptide growth factors (GFs) (Giannobile, W.V. *Bone 19*(1)Suppl.:23S-37S, 1996); and fibronectin and factor XIII (Grinnell, F. *J. Cell. Biochem. 26*:107-16, 1984).

Although the above therapeutic agents have been provided for the purposes of illustration, it should be understood that the present disclosure is not so limited. For example, although agents are specifically referred to above, the present invention should be understood to include analogues, derivatives and conjugates of such agents. As an illustration, paclitaxel should be understood to refer to not only the common chemically available form of paclitaxel, but analogues (*e*.*g*., taxotere, as noted above) and paclitaxel conjugates *(e.g.,* paclitaxel-PEG, paclitaxel-dextran or paclitaxel-xylos). In addition, as will be evident to one of skill in the art, although the agents set forth above may be noted within the context of one class, many of the agents listed in fact have multiple biological activities. Further, more than one therapeutic agent may be utilized at a time (*i.e*., in combination), or delivered sequentially.

### POLYMERIC CARRIERS

As noted above, therapeutic compositions of the present invention may additionally comprise a polymeric carrier. A wide variety of polymeric carriers may be utilized to contain and or delivery one or more of the therapeutic agents discussed above, including for example both biodegradable and non-biodegradable compositions. Representative examples of biodegradable compositions include albumin, collagen, gelatin, starch, cellulose (methylcellulose, hydroxypropylcellulose, hydroxypropylmethylcellulose, carboxymethylcellulose, cellulose acetate phthalate, cellulose acetate succinate, hydroxypropylmethylcellulose phthalate), casein, dextrans, polysaccharides, poly(caprolactone), fibrinogen, poly(D,L lactide), poly(D,L-lactide-co-glycolide), poly(glycolide), poly(hydroxybutyrate), poly(alkylcarbonate) and poly(orthoesters), polyesters, poly(hydroxyvaleric acid), polydioxanone, poly(ethylene terephthalate), poly(malic acid), poly(tartronic acid), polyanhydrides, polyphosphazenes, poly(amino acids and their copolymers *(see generally* Illium, L., Davids, S.S. (eds.) ''Polymers in controlled Drug Delivery" Wright, Bristol, 1987; Arshady, *J. Controlled Release 17*:1-22, 1991; Pitt, *Int. J. Phar. 59*:173-196, 1990; Holland et al., *J. Controlled Release 4*:155-0180, 1986). Representative examples of nondegradable polymers include EVA copolymers, silicone rubber, acrylic polymers (polyacrylic acid), poly(methylacrylic acid), poly(methylmethacrylate), poly(hydroxyethylmethacrylate), poly(alkylcynoacrylate), poly(ethylene), poly(proplene), polyamides (nylon 6,6), poly(urethane), poly(ester urethanes), poly(ether urethanes), poly(carbonate urethanes), poly(ester-urea), polyethers [poly(ethylene oxide), poly(propylene oxide), pluronics, poly(tetramethylene glycol)], silicone rubbers and vinyl polymers [polyvinylpyrrolidone, poly(vinyl alcohol), poly(vinyl acetate phthalate). Polymers may also be developed which are either anionic *[e.g.,* alginate, carrageenin, caboxymethyl cellulose and poly(acrylic acid)], or cationic *[e.g.,* Chitosan, poly-1-lysine, polyethylenimine, and poly (allyl amine)] *(see generally,* Dunn et al., *J. Applied Polymer Sci. 50*:353-365, 1993; Cascone et al., *J. Materials Sci.: Materials in Medicine 5*:770-774, 1994; Shiraishi et al., *Biol. Pharm. Bull. 16*(11):1164-1168, 1993; Thacharodi and Rao, *Int'l J. Pharm. 120*:115-118, 1995; Miyazaki et al., *Int'l J. Pharm. 118*:257-263, 1995). Particularly preferred polymeric carriers include poly(ethylene-vinyl acetate), poly (D,L-lactic acid) oligomers and polymers, poly(L-lactic acid) oligomers and polymers, poly(glycolic acid), copolymers of lactic acid and glycolic acid, poly(caprolactone), copolymers of poly(lactic acid) or poly(glycolic acid) and poly(caprolactone), poly(valerolactone), poly(anhydrides), copolymers of poly(caprolactone) or poly(lactic acid) with polyethylene glycol and blends thereof.

Polymeric carriers can be fashioned in a variety of forms, with desired release characteristics and/or with specific desired properties. For example, polymeric carriers may be fashioned to release a therapeutic agent upon exposure to a specific triggering event such as pH *(see, e.g.,* Heller et al., "Chemically Self-Regulated Drug Delivery Systems," in *Polymers in Medicine III*, Elsevier Science Publishers B.V., Amsterdam, 1988, pp. 175-188; Kang et al., *J. Applied Polymer Sci.* 48:343-354, 1993; Dong et al., *J. Controlled Release 19:171-178, 1992;* Dong and Hoffman, *J. Controlled Release 15*:141-152, 1991; Kim et al., *J. Controlled Release 28*:143-152, 1994; Comejo-Bravo et al., *J. Controlled Release 33*:223-229, 1995; Wu and Lee, *Pharm. Res. 10*(10):1544-1547, 1993; Serres et al., *Pharm. Res. 13*(2):196-201, 1996; Peppas, "Fundamentals of pH- and Temperature-Sensitive Delivery Systems," in Gurny et al. (eds.), *Pulsatile Drug Delivery,* Wissenschaftliche Verlagsgesellschaft mbH, Stuttgart, 1993, pp. 41-55; Doelker, "Cellulose Derivatives," 1993, in Peppas and Langer (eds.), *Biopolymers I,* Springer-Verlag, Berlin). Representative examples of pH-sensitive polymers include poly(acrylic acid) and its derivatives (including for example, homopolymers such as poly(aminocarboxylic acid); poly(acrylic acid); poly(methyl acrylic acid)), copolymers of such homopolymers, and copolymers of poly(acrylic acid) and acrylmonomers such as those discussed above. Other pH sensitive polymers include polysaccharides such as cellulose acetate phthalate; hydroxypropylmethylcellulose phthalate; hydroxypropylmethylcellulose acetate succinate; cellulose acetate trimellilate; and chitosan. Yet other pH sensitive polymers include any mixture of a pH sensitive polymer and a water soluble polymer.

Likewise, polymeric carriers can be fashioned which are temperature sensitive *(see, e.g.,* Chen et al., "Novel Hydrogels of a Temperature-Sensitive Pluronic Grafted to a Bioadhesive Polyacrylic Acid Backbone for Vaginal Drug Delivery," in *Proceed. Intern. Symp. Control. Rel. Bioact. Mater. 22*:167-168, Controlled Release Society, Inc., 1995; Okano, "Molecular Design of Stimuli-Responsive Hydrogels for Temporal Controlled Drug Delivery," in *Proceed. Intern. Symp. Control. Rel. Bioact. Mater.* 22:111-112, Controlled Release Society, Inc., 1995; Johnston et al., *Pharm. Res. 9*(3):425-433, 1992; Tung, *Int'l J. Pharm. 107*:85-90, 1994; Harsh and Gehrke, *J. Controlled Release 17*:175-186, 1991; Bae et al., *Pharm. Res. 8*(4):531-537, 1991; Dinarvand and D'Emanuele, *J. Controlled Release 36*:221-227, 1995; Yu and Grainger, "Novel Thermo-sensitive Amphiphilic Gels: Poly N-isopropylacrylamide-co-sodium acrylate-co-n-N-alkylacrylamide Network Synthesis and Physicochemical Characterization," Dept. of Chemical & Bioligal Sci., Oregon Graduate Institute of Science & Technology, Beaverton, OR, pp. 820-821; Zhou and Smid, "Physical Hydrogels of Associative Star Polymers," Polymer Research Institute, Dept. of Chemistry, College of Environmental Science and Forestry, State Univ. of New York, Syracuse, NY, pp. 822-823; Hoffman et al., "Characterizing Pore Sizes and Water 'Structure' in Stimuli-Responsive Hydrogels," Center for Bioengineering, Univ. of Washington, Seattle, WA, p. 828; Yu and Grainger, "Thermo-sensitive Swelling Behavior in Crosslinked N-isopropylacrylamide Networks: Cationic, Anionic and Ampholytic Hydrogels," Dept. of Chemical & Biological Sci., Oregon Graduate Institute of Science & Technology, Beaverton, OR, pp. 829-830; Kim et al., *Pharm. Res. 9*(3):283-290, 1992; Bae et al., *Pharm. Res. 8*(5):624-628, 1991; Kono et al., *J. Controlled Release 30*:69-75, 1994; Yoshida et al., *J. Controlled Release 32*:97-102, 1994; Okano et al., *J. Controlled Release 36*:125-133, 1995; Chun and Kim, *J. Controlled Release 38*:39-47, 1996; D'Emanuele and Dinarvand, *Int'l J*., *Pharm. 118*:237-242, 1995; Katono et al., *J. Controlled Release 16*:215-228, 1991; Hoffman, "Thermally Reversible Hydrogels Containing Biologically Active Species," in Migliaresi et al. (eds.), *Polymers in Medicine III*, Elsevier Science Publishers B.V., Amsterdam, 1988, pp. 161-167; Hoffman, "Applications of Thermally Reversible Polymers and Hydrogels in Therapeutics and Diagnostics," in *Third International Symposium on Recent Advances in Drug Delivery Systems,* Salt Lake City, UT, Feb. 24-27, 1987, pp. 297-305; Gutowska et al., *J. Controlled Release 22*:95-104, 1992; Palasis and Gehrke, *J. Controlled Release 18*:1-12, 1992; Paavola et al., *Pharm. Res. 12*(12):1997-2002, 1995).

Representative examples of thermogelling polymers include homopolymers such as poly(N-methyl-N-n-propylacrylamide), LCST=19.8°C; poly(N-n-propylacrylamide), 21.5; poly(N-methyl-N-isopropylacrylanude), 22.3; poly(N-n-propylmethacrylamide), 28.0; poly(N-isopropylacrylamide), 30.9; poly(N, n-diethylacrylamide), 32.0; poly(N-isopropylmethacrylamide), 44.0; poly(N-cyclopropylacrylamide), 45.5; poly(N-ethylmethyacrylamide), 50.0; poly(N-methyl-N-ethylacrylamide), 56.0; poly(N-cyclopropylmethacrylamide), 59.0; poly(N-ethylacrylamide), 72.0. Moreover thermogelling polymers may be made by preparing copolymers between (among) monomers of the above, or by combining such homopolymers with other water soluble polymers (*e*.*g*., poly(acrylic acid), poly(methylacrylic acid), poly(acrylate), poly(butyl methacrylate), poly(acrylamide) and poly(N-n-butyl acrylamide) and derivatives thereof.

Other representative examples of thermogelling polymers include cellulose ether derivatives such as hydroxypropyl cellulose, 41°C; methyl cellulose, 55°C; hydroxypropylmethyl cellulose, 66°C; and ethylhydroxyethyl cellulose, and pluronics such as F-127, 10 - 15°C; L-122, 19°C; L-92, 26°C; L-81, 20°C; and L-61, 24°C.

A wide variety of forms may be fashioned by the polymeric carriers of the present invention, including for example, rod-shaped devices, pellets, slabs or capsules *(see, e*.*g*., Goodell et al., *Am*. *J*. *Hosp*. *Pharm*. *43*:1454-1461, 1986; Langer et al., "Controlled release of macromolecules from polymers", in *Biomedical polymers, Polymeric materials and pharmaceuticals for biomedical use*, Goldberg, E.P., Nakagim, A. (eds.) Academic Press, pp. 113-137, 1980; Rhine et al., *J*. *Pharm*. *Sci*. *69*:265-270, 1980; Brown et al., *J*. *Pharm*. *Sci*. *72*:1181-1185, 1983; and Bawa et al., *J*. *Controlled Release 1*:259-267, 1985). Therapeutic agents may be linked by occlusion in the matrices of the polymer, bound by covalent linkages, or encapsulated in microcapsules. Within certain preferred embodiments of the invention, therapeutic compositions are provided in non-capsular formulations such as microspheres (ranging from nanometers to micrometers in size preferably having an average size of between 0.5 and 200 µm), pastes, threads of various size, films, particulates, gels, foams, molds and sprays.

Preferably, therapeutic compositions of the present invention are fashioned in a manner appropriate to the intended use. Within certain aspects of the present invention, the therapeutic composition should be biocompatible; and release one or more therapeutic agents over a period of several days to months. For example, "quick release" or "burst" therapeutic compositions are provided that release greater than 10%; 20%, or 25% (w/v) of a therapeutic agent (*e*.*g*., paclitaxel) over a period of 7 to 10 days. Such "quick release" compositions should; within certain embodiments, be capable of releasing chemotherapeutic levels (where applicable) of a desired agent. Within other embodiments, "slow release" therapeutic compositions are provided that release less than 10% (w/v) of a therapeutic agent over a period of 7 to 10 days. Further, therapeutic compositions of the present invention should preferably be stable for several months and capable of being produced and maintained under sterile conditions.

Within certain aspects of the present invention, therapeutic compositions may be fashioned in any size ranging from 50 nm to 500 µm, depending upon the particular use. Alternatively, such compositions may also be readily applied as a "spray", which solidifies into a film or coating. Such sprays may be prepared from microspheres of a wide array of sizes, including for example, from 0.1 µm to 3 µm, from 10 µm to 30 µm, and from 30 µm to 100 µm.

Within other certain aspects of the present invention, therapeutic compositions may be readily applied as a "spray" solution which solidifies into a film or coating. Such sprays may be prepared by incorporating the therapeutic agents into any of the above-identified carriers (polymeric or non-polymeric).

Therapeutic compositions of the present invention may also be prepared in a variety of "paste" or gel forms. For example, within one embodiment of the invention, therapeutic compositions are provided which are liquid at one temperature (*e.g.*, temperature greater than 37°C), and solid or semi-solid at another temperature (*e.g.*, ambient body temperature, or any temperature lower than 37°C). Such "thermopastes" may be readily made given the disclosure provided herein.

Within yet other aspects of the invention, the therapeutic compositions of the present invention may be formed as a film. Preferably, such films are generally less than 5, 4, 3, 2, or 1, mm thick, more preferably less than 0.75 nun or 0.5 mm thick, and most preferably less than 500 µm to 50 µm thick. Such films are preferably flexible with a good tensile strength (*e*.*g*., greater than 50, preferably greater than 100, and more preferably greater than 150 or 200 N/cm²), good adhesive properties (*i*.*e*., readily adheres to moist or wet surfaces), and have controlled permeability.

Within certain embodiments of the invention, the therapeutic compositions may also comprise additional ingredients such as surfactants (e.g. pluronics such as F-127, L-122, L-92, L-81, and L-61).

Within further aspects of the present invention, polymeric carriers are provided which are adapted to contain and release a hydrophobic compound, the carrier containing the hydrophobic compound in combination with a carbohydrate, protein or polypeptide. Within certain embodiments, the polymeric carrier contains or comprises regions, pockets, or granules of one or more hydrophobic compounds. For example, within one embodiment of the invention, hydrophobic compounds may be incorporated within a matrix which contains the hydrophobic compound, followed by incorporation of the matrix within the polymeric carrier. A variety of matrices can be utilized in this regard, including for example, carbohydrates and polysaccharides such as starch, cellulose, dextran, methylcellulose, and hyaluronic acid, proteins or polypeptides such as albumin, collagen and gelatin. Within alternative embodiments, hydrophobic compounds may be contained within a hydrophobic core, and this core contained within a hydrophilic shell. For example, as described within the Examples, paclitaxel may be incorporated into a hydrophobic core (*e.g.*, of the poly D,L lactic acid-PEG or MePEG aggregate) which has a hydrophilic shell.

A wide variety of hydrophobic compounds may be released from the polymeric carriers described above, including for example: certain hydrophobic compounds which disrupt microtubule function such as paclitaxel and estramustine; hydrophobic proteins such as myelin basic protein, proteolipid proteins of CNS myelin, hydrophobic cell wall protein, porins, membrane proteins *(EMBO J. 12*(9):3409-3415, 1993), myelin oligodendrocyte glycoprotein ("MOG") (*Biochem. and Mol. Biol. Int. 30*(5):945-958, 1993, P27 *Cancer Res. 53*(17):4096-4101, 1913, bacterioopsin, human surfactant protein ("HSB"; *J. Biol. Chem. 268*(15):11160-11166, 1993), and SP-B or SP-C *(Biochimica et Biophysica Acta* 1105(1):161-169, 1992).

Representative examples of the incorporation of therapeutic agents such as those described above into a polymeric carriers to form a therapeutic composition, is described in more detail below in the Examples.

### OTHER CARRIERS

Other carriers that may likewise be utilized to contain and deliver the therapeutic agents described herein include: hydroxypropyl β cyclodextrin (Cserhati and Hollo, *Int. J*. *Pharm*. *108*:69-75, 1994), liposomes (see *e.g.,* Sharma et al., *Cancer Res. 53*:5877-5881, 1993; Sharma and Straubinger, *Pharm*. *Res. 11*(60):889-896, 1994; WO 93/18751; U.S. Patent No. 5,242,073), liposome/gel (WO 94/26254), nanocapsules (Bartoli et al., *J*. *Microencapsulation 7*(2):191-197, 1990), micelles (Alkan-Onyuksel et al., *Pharm. Res. 11*(2):206-212, 1994), implants (Jampel et al., *Invest*. *Ophthalm*. *Vis*. *Science 34*(11):3076-3083, 1993; Walter et al., *Cancer Res. 54*:22017-2212, 1994; U.S. Patent No. 4,882,168), nanoparticles (with or without surface modification) (Violante and Lanzafame PAACR; U.S. Patent No. 5,145,684; U.S. Patent No. 5,399,363), emulsion/solution (U.S. Patent No. 5,407,683), micelle (surfactant) (U.S. Patent No. 5,403,858), synthetic phospholipid compounds (U.S. Patent No. 4,534,899), gas borne dispersion (U.S. Patent No. 5,301,664), liquid emulsions, foam spray, gel lotion cream, ointment, dispersed vesicles, particles or droplets solid- or liquid - aerosols, microemulsions (U.S. Patent No. 5,330,756), polymeric shell (nano- and microcapsule) (U.S. Patent No. 5,439,686), taxoid-based compositions in a surface-active agent (U.S. Patent No. 5,438,072), emulsion (Tarr et al., *Pharm Res. 4*:62-165, 1987), and nanospheres (Hagan et al., *Proc. Intern. Symp. Control Rel. Bioact. Mater. 22,* 1995; Kwon et al., *Pharm Res. 12*(2):192-195; Kwon et al., *Pharm Res. 10*(7):970-974; Yokoyama et al., *J. Contr. Rel. 32*:269-277, 1994; Gref et al., *Science 263*:1600-1603, 1994; Bazile et al., *J Pharm. Sci. 84*:493-498, 1994).

As discussed in more detail below, therapeutic agents of the present invention, which are optionally incorporated within one of the carriers described herein to form a therapeutic composition, may be prepared and utilized to treat or prevent a wide variety of conditions.

### TREATMENT OR PREVENTION OF COMPROMISED BODY PASSAGEWAY OR CAVITY INTEGRITY

As noted above, the present invention relates generally to compositions for improving the integrity of body passageways or cavities following surgery or injury, and more specifically, to compositions comprising therapeutic agents which may be delivered to the external walls of body passageways or cavities for the purpose of strengthening the walls of the passageway or cavity, including, for example, iatrogenic complications of arterial and venous catheterization, vascular and aortic dissection, cardiac rupture, aneurysm, cardiac valve dehiscence, passageway rupture and surgical wound repair.

In order to further the understanding of such conditions, representative complications leading to compromised body passageway or cavity integrity are discussed in more detail below.

### A. Iatrogenic Complications of Arterial and Venous Catheterization

Utilizing the agents, compositions and methods provided herein, iatrogenic complications of arterial and venous catheterization can be readily prevented or treated. For example, within one embodiment of the invention these complications may be prevented by delivering to the adventitial surface of the vessel into which the sheath was introduced an agent that stabilizes microtubules and/or a polymeric carrier.

Iatrogenic arterial and venous injuries represent the most common type of vascular trauma in most hospitals. A major trauma, these injuries frequently require surgical repair. Some patients have long term limb dysfunction after the vascular injury, and other complications can include local neuralgias, claudication and limb loss.

The types of vascular complications have changes over the years, with a decrease in arterial thrombosis and an increase in the number of hematomas and pseudoaneurysms. This change is due primarily to large catheter sheaths, thrombolytic agents and anticoagulants, and longer duration of catheter use.

Despite measures to reduce complications, hematomas and pseudoaneurysms can form following femoral catheterization (*e.g*., balloon angioplasty, atherectomy). These generally start immediately, within 12 hours or as patients begin to move the limb. If a pseudoaneurysm is confirmed, ultrasound-guided compression can be applied to initially treat the area. Surgery is still required to repair between 20% and 30% of femoral catheter injuries, oftentimes requiring general anesthesia and invasive procedures.

Ongoing uncontrolled hemorrhage after catheter removal affects about 10% to 15% of patients with catheter injuries. An arteriovenous fistula usually occurs after a low groin puncture and involves the deep or superficial femoral arteries and their adjacent veins. Large chronic or symptomatic arteriovenous fistulae require surgical repair, while small asymptomatic fistulae can be left untreated in many patients. Arterial dissection usually occurs in patients with underlying aortoiliac atherosclerosis and tortuous pelvic arteris.

Arterial and venous complications also occur with translumbar aortography, brachial artery catheterization, transaxillary arterial catheters, intra-aortic balloon pumps, radial artery catheters, subclavian vein catheters, jugular vein catheters and pulmonary artery catheters. Vascular problems include pseudoaneurysm, vessel dissection, hemorrhage and arterivenous fistulae.

In order to prevent the complications associated with arterial and venous catheterization, such as those discussed above, a wide variety of therapeutic agents (with or without a carrier) or polymers may be delivered to the external portion of the blood vessel *via* the adventitia of the blood vessel. The polymer or therapeutic agent/polymer complex would be applied to the external portion of the vessel following the interventional or surgical procedure in order to prevent complications. The purpose of applying these entities to the outside of the blood vessel is to induce or stimulate the formation of a connective tissue layer which would provide added stability and improve the integrity of the vessel wall, thereby preventing the associated complications of catheterization.

Particularly preferred therapeutic agents include microtubule stabilizing agents, fibrosis inducers, angiogenic factors, growth factors and cytokines and other factors involved in the wound healing or fibrosis cascade.

### B. Pseudoaneurysms

Utilizing the agents, compositions and methods provided herein, pseudoaneuryms can be readily prevented or treated. For example, within one embodiment of the invention these complications may be prevented by delivering to the adventitial surface of the injured vessel an agent that stabilizes microtubules and/or a polymeric carrier.

A pseudoaneurysm is a pulsatile hematoma that communicates with an artery through a disruption in the arterial wall. It can result from an infection, trauma and surgical procedures (Vascular Surgery, 4th Edition. Philadelphia, PA, W.B. Saunders Company, 1995). All pseudoaneurysms disrupt the continuity of an artery with blood extravasation into surrounding tissues, resulting in a fibrous tissue capsule that enlarges progressively due to arterial pressure.

The cause of all pseudoaneurysms is a disruption in arterial continuity and this can be due to many factors including arterial trauma, infection, vasculitis, complications due to vascular surgery leading to anastomotic separation and diagnostic and therapeutic procedures involving arterial puncture.

Iatrogenic pseudoaneurysms arise as a consequence of arterial reconstructions. The strength of the anastomosis and the union between the artery and the vascular graft is dependent upon the integrity and durability of the suture material. Tissue ingrowth alone is inadequate to provide the required strength regardless of the extent of soft tissue incorporation of vascular grafts (Kottmeir & Wheat, *Am. J. Surg., 31(2)*:128, 1965). Anastomotic pseudoaneurysm formation is also thought to be due to factors such as differences in compliance between native and graft materials, shearing forces along anastomotic lines, vibratory fatigue, graft position and anticoagulation; all these factors allow blood to leave the vessel via partial dehiscence of the suture line. Since prosthetic graft materials have less compliance than native arteries, a dilatation of the artery occurs, thus inducing disruptive stress on the anastomosis (Vascular Surgery: Principles and Techniques, 3^{rd} Edition. Norwalk, CT, Appleton and Lange, 1989).

The loss in structural integrity is due to suture material fatigue, prosthesis degeneration and host vessel degeneration independent of its relationship to the prosthetic material. Decreased elasticity is due to fibrous degeneration and inhibits arterial adaptability to mechanical stresses. Other factors leading to host vessel degeneration include the progression of atherosclerosis and local factors that accelerate degeneration, such as perigraft fluid collections, excessive endarterectomy and extensive artery mobilization during the initial procedure (Vascular Surgery, 4th Edition. Philadelphia, PA, W.B. Saunders Company, 1995).

Pseudoaneurysms may also be caused by graft infections following bypass procedures. *Staphylococcus epidermidis* or other coagulase-negative staphylococcal species, are common infectious organisms. Cytolysins from these organisms cause disincorporation of the graft from host tissues and increase the likelihood of pseudoaneurysm formation.

Pseudoaneurysms can form from angiography and thrombolytic therapy. Interventional procedures, such as percutaneous transluminal angioplasty, that use larger catheters and aggressive manipulations have a greater incidence of complications than simple diagnostic procedures.

Infectious pseudoaneurysms result from septic emboli, contiguous infection and intravenous drug abuse, and occur most commonly in the groin, neck and upper extremities. Other causes of pseudoaneurysms are blunt and penetrating injuries with the former common in the popliteal artery and distal upper extremity arteries, the latter in the more superficial femoral and carotid vessels. Vasculitides is also associated with pseudoaneurysm formation. These pseudoaneurysms are much more common in those vasculitides that involve the larger arteries.

The clinical manifestation of most pseudoaneurysms includes local symptoms, such as pain, rapid expansion or venous obstruction associated with a palpable mass. The most common site of presentation is the groin. An anastomotic aneurysm presents itself on average within 6 years, with a range of 2.5 months to 19 years, of surgery. Earlier presentation is correlated with infection or a second procedure performed upon the same anatomic area.

Pseudoaneurysms can form in a variety of blood vessels. These are discuss below.

***Aortic pseudoaneurysms*** are relatively rare and difficult to diagnose due to their location. The scarcity of symptoms associated with intra-abdominal pseudoaneurysms prior to catastrophic complications further increases the problem. Acute thrombosis of aortic pseudoaneurysms is seen in 25 per cent of patients. Renovascular hypertension and distal embolization may be evident, and other more fatal complications include acute retroperitoneal or abdominal hemorrhage. Pseudoaneurysms of the abdominal aorta are rare (less than 2 per cent of all pseudoaneurysms), but occur in association with aortic aneurysm repairs.

***Iliac pseudoaneurysms*** are difficult to diagnose and manifest when thrombosis or distal embolization has occurred. They are commonly associated with aortoiliac bypass procedures and occur less frequently as a consequence of trauma. When these pseudoaneurysms occur after trauma to the pelvis they may be associated with pelvic abscesses (Landrenau & Snyder, *Am. J*. *Surg., 163*:197, 1992). Operative repair is challenging especially in the presence of sepsis, and iliac artery ligation and extra-anatomic bypass are recommended if primary repair is impossible. Symptoms are a consequence of encroachment from the ureters, bladder, sacral plexus and iliac veins.

***Femoral pseudoaneurysms*** are the most common and account for more than three fourths of all clinically important pseudoaneurysms; these are commonly caused by disruption of a prosthetic arterial anastomosis. This occurs in 1.5% to 3% of patients undergoing either aortofemoral or femoropopliteal bypass grafting (Hollier et al., *Ann. Surg., 191(16)*:715, 1979). If untreated, these pseudoaneurysms result in vessel thrombosis, distal embolization or rupture. Early diagnosis and treatment is standard care since elective repairs of pseudoaneurysms have lower morbidity and mortality rates and higher long-term patency rates. In most cases, placement of an interposition conduit, composed of either prosthetic material or saphenous vein, is the preferred procedure as it results in less than 4% mortality and greater than 75% patency rate. Another less common cause of femoral pseudoaneurysm is femoral artery catheterization. The incidence of this complication ranges from 0.05% to 2.0% of all femoral artery catheter procedures. The incidence may be increased due to hypertension, anticoagulation, multiple punctures, the use of large-bore catheters and sheaths and the cannulation of poorly compliant or calcific vessels. Operative intervention is necessary if the pseudoaneurysm is symptomatic, expanding, associated with an extremely large hematoma, or persists for more than 6 weeks (Vascular Surgery, 4th Edition. Philadelphia, PA, W.B. Saunders Company, 1995).

***Popliteal pseudoaneurysms*** are less common than true popliteal aneurysms and they account for about 3% of all pseudoaneurysms. Blunt trauma may result in pseudoaneurysm formation in the popliteal area, depending on the degree of tethering of the vessel above and below the knee joint. Distal peripheral arterial pseudoaneurysms commonly occur due to catheter placement for continuous arterial pressure monitoring. Treatment consists of excision of the pseudoaneurysm with ligation or interposition vein graft placement.

***Carotid pseudoaneurysms*** formation is rarely associated with carotid endarterectomy. Incidence ranges from 0.15% to 0.06%, and symptoms generally occur 4 to 6 months following operation and may include a painful pulsatile cervical mass, transient ischemic attacks secondary to emboli and hoarseness due to recurrent laryngeal nerve compression. Differential diagnosis includes chemodectoma of the carotid body, lymphadenopathy and kinking of an endarterectomized carotid artery. Most patients with pseudoaneurysms following carotid endarterectomy should undergo operative correction to eliminate risk of embolization. Interposition grafting is the preferred method of treatment since ligation of the carotid artery is associated with at least a 20% incidence of major stroke. Difficulty in dissection may be due to the presence of scar and problems with identification of important neural structure, such as the vagus and hypoglossal nerves. If the pseudoaneurysm involves the carotid bifurcation and the defect is small and no evidence of infection exists, then primary closure may be possible. If a large defect is present, patch angioplasty is indicated with either prosthetic material or saphenous vein. If the bifurcation has completely degenerated, a bypass graft from the more proximal common carotid artery to the internal carotid artery with a reversed saphenous vein graft is necessary. Treatment consists of either ligation or replacement of a prosthetic patch with autogenous vein. An extracranial-intracranial bypass may be performed if cerebral ischemia occurs. Primary mycotic carotid pseudoaneurysms are rare but they are associated with lethal complications. An aneurysmal abscess or pseudoaneurysm should be suspected in any drug-abusing patient with a painful neck mass and cellulitis. The common carotid artery is involved in most lesions rather than the internal carotid artery. Severe cellulitis can be treated with antibiotics, at which time ligation of the involved artery and evacuation of infected hematoma is performed. Rarely is bypass grafting feasible (Vascular Surgery, 4th Edition. Philadelphia, PA, W.B. Saunders Company, 1995).

In order to prevent the formation of pseudoaneurysms, such as those discussed above, a wide variety of therapeutic agents (with or without a carrier) or polymers may be delivered to the external portion of the blood vessel *via* the adventitia of the blood vessel. The polymer or therapeutic agent/polymer complex would be applied to the external portion of the vessel following the interventional or surgical procedure in order to prevent the formation of the pseudoaneurysm. The purpose of applying these entities to the outside of the blood vessel is to induce or stimulate the formation of a connective tissue layer which would provide added stability and improve the integrity of the vessel wall, thereby preventing the formation of the pseudoaneurysm.

Particularly preferred therapeutic agents include microtubule stabilizing agents, fibrosis inducers, angiogenic factors, growth factors and cytokines and other factors involved in the wound healing or fibrosis cascade.

### C. Cardiac Rupture

Utilizing the agents, compositions and methods provided herein, cardiac rupture can be readily treated or prevented. For example, within one embodiment of the invention these complications may be treated by delivering to the outer surface of the heart an agent that stabilizes microtubules and/or a polymeric carrier.

The incidence of cardiac rupture following myocardial infarction ranges between 4% and 24% (Padro et al., *Ann. Thor. Surg., 55*:20-24, 1993), and is considered to cause more than 25,000 deaths a year in the United States.

In order to prevent or treat cardiac rupture, a wide variety of therapeutic agents (with or without a carrier) or polymers may be delivered to the compromised surface of the heart so as to stimulate the formation of connective tissue and improve the integrity of the wall. The polymer or therapeutic agent/polymer complex would be applied to the surface following surgical repair of the rupture or, alternatively, through a minimally invasive procedure whereby the material is injected onto the compromised surface. The purpose of applying these entities to the heart wall is to induce or stimulate the formation of a connective tissue layer which would provide added stability and improve the integrity of the wall, thereby preventing cardiac rupture.

Particularly preferred therapeutic agents include microtubule stabilizing agents, fibrosis inducers, angiogenic factors, growth factors and cytokines and other factors involved in the wound healing or fibrosis cascade.

### D. Periprosthetic Leaks and Heart Valve Dehiscence

Utilizing the agents, compositions and methods provided herein, periprosthetic leaks and valve dehiscence can be readily prevented or treated. For example, within one embodiment of the invention these complications may be prevented by delivering to the periphery of the annular ring an agent that stabilizes microtubules and/or a polymeric carrier.

In order to prevent periprosthetic leaks and/or heart valve dehiscence, a wide variety of therapeutic agents (with or without a carrier) or polymers may be delivered to the external portion of the valve. The polymer or therapeutic agent/polymer complex would be applied to the external portion of the valve following valve replacement surgery in order to prevent periprosthetic leaks and/or heart valve dehiscence. The purpose of applying these entities to the outside of the valve is to induce or stimulate the formation of a connective tissue layer which would provide added stability and improve the integrity of the cardiac wall, thereby preventing the complications that lead to periprosthetic leaks and/or heart valve dehiscence.

Particularly preferred therapeutic agents include microtubule stabilizing agents, fibrosis inducers, angiogenic factors, growth factors and cytokines and other factors involved in the wound healing or fibrosis cascade.

### E. Vascular Surgical Procedures

Utilizing the agents, compositions and methods provided herein, complication following vascular surgery can be readily prevented or treated. For example, within one embodiment of the invention a microtubule stabilizing agent, such as paclitaxel, may be applied to the adventitial surface of a repaired blood vessel in order to increase the strength of the vascular wound.

Repaired blood vessels have a diminished strength which can lead to leakage or aneurysm formation. Within one embodiment of the present invention, a method for increasing the strength of repaired vessels to values similar to those of noninjured blood vessels is described.

For example, after repair of a vascular wound, a thin film composed of poly(ethylene vinyl acetate) is wrapped around the repaired blood vessel so that the entire wound is covered. The film can be sutured or glued in place. The treated wound can be an anastomosis between a blood vessel and a vascular graft, an anastomosis between two blood vessels or an incision in an artery or vein. The presence of the film promotes perivascular tissue growth in between the film and the vessel within two weeks. This new tissue dramatically increases the strength of the repaired vessel to values similar to those of noninjured vessels.

In order to increase vascular wound strength, a wide variety of therapeutic agents (with or without a carrier) or polymers may be delivered to the external portion of the blood vessel via the adventitia of the blood vessel. The polymer or therapeutic agent/polymer complex would be applied to the external portion of the vessel following the surgical procedure. The purpose of applying these materials to the outside of the blood vessel is to induce or stimulate the formation of a connective tissue layer which would provide added stability and improve the integrity of the vessel wall, thereby preventing leakage or aneurysm formation.

Particularly preferred therapeutic agents include microtubule stabilizing agents, fibrosis inducers, angiogenic factors, growth factors and cytokines and other factors involved in the wound healing or fibrosis cascade.

### F. Aneurysms

Utilizing the agents, compositions and methods provided herein, aneurysms can be readily prevented or treated. For example, within one embodiment of the invention a microtubule stabilizing agent, such as paclitaxel, may be applied to the adventitial surface of the compromised blood vessel in order to increase the strength of the vascular wall.

An aneurysm is a widening of a vessel involving the stretching of fibrous tissue within the media of the vessel. A widening of the vessel is considered a true aneurysm, whereas a false aneurysm is a localized rupture of the artery with sealing over by clot or adjacent structures. Aneurysms have a tendency to enlarge and as the radius increases so does the wall tension. An aneurysm is defined as a permanent localized dilatation of an artery, with a greater than 50 percent increase in diameter compared to the normal diameter of the artery. The diagnosis of an aneurysm depends on a comparison of the aortic diameter of the suspicious area with that of the normal area of artery above the dilatation (Santilli, 1997).

Aneurysms can be classified according to cause, morphology and location. The most common cause is atherosclerosis, other causes include cystic medical necrosis, trauma, and infection. Rarer causes are rheumatic aortitis, Takayasu's syndrome, temporal arteritis, and relapsing polychondritis. There are three morphological types of aneurysms: (1) fusiform, in which the aneurysm encompasses the entire circumference of the aorta and assumes a spindle shape; (2) saccular, in which only a portion of the circumference is involved and in which there is a neck and an asymmetric outpouching of the aneurysm; (3) dissecting, in which an intimal tear permits of column of blood to dissect along the media of the vessel. Aneurysms are also classified by location, involving (1) the ascending aorta, including the sinuses of the Valsalva; (2) the aortic arch; (3) the descending thoracic aorta, originating just distal to the left subclavian artery; and (4) the abdomen, most commonly distal to the renal arteries (Cohen, 1996).

Abdominal aortic aneurysms are a localized dilatation of the abdominal aorta, most commonly found in the infrarenal portion of the abdominal aorta. They occur in 5 to 7 percent of people over the age of 60 years in the United States (Santilli, 1997).

Inflammation is a prominent feature of abdominal aortic aneurysms with infiltrating macrophages and lymphocytes scattered throughout the intima/plaque and adventitia. The lymphocytes present in abdominal aortic aneurysm tissue are T- and B-cells, and adventitial inflammation is a consistent feature of this type of aneurysm. The term "inflammatory aneurysm" represents an extreme of the periadventitial inflammation found in all abdominal aortic aneurysms (Grange, 1997).

For aneurysms to enlarge, the collagen and elastin matrix fibers of the aortic media must be degraded first. Degradation of the extracellular matrix and loss of structural integrity of the aortic wall have been extensively researched. Increased collagenase and elastase activity has been documented in aortic aneurysms, with the greatest increases in rapidly enlarging and ruptured aneurysms. Inflammatory cells may play an important role in the local release of proteolytic enzymes, particularly metalloproteinases. Experimental enzymatic destruction of the medial lammelar architecture of the aorta results in aneurysm formation with dilatation and rupture (Zarins, 1997).

There is an extensive loss of medial elastin in aneurysms but this appears not to have a major effect on the overall mechanical strength of the aortic wall. It is speculated that ongoing destruction, synthesis, and reorganization of adventitial collagen is more important in the progression of aneurysmal dilatation and subsequent rupture. Another prominent feature, is an inflammatory infiltrate of mononuclear cells at the junction between the adventitia and the media. Whereas macrophages are present in both aneurysmal and occlusive aortas, T lymphocytes are infrequent in the adventitia of normal of occlusive vessels. Lymphocytes are known to secrete gamma interferon, tumor necrosis factor-α (TNF-α), and interleukin-2 (IL-2), which increases macrophage proteolytic activity and, therefore, may be important in the pathogenesis of aneurysm disease. Macrophages are a potential source of matrix metalloproteinases and various cytokines. These infiltrating macrophages and lymphocytes may be involved in the destruction of the aortic matrix (Tilson, 1997).

Mesenchymal cells of the aorta may also play a role in aneurysm development. The smooth muscle cells in the adventitia of inflammatory aneurysms have been found to be abundant in rough endoplasmic reticulum. These smooth muscle cells may be involved in matrix deposition and production of enzymes responsible for its destruction.

Tilson (1997) has proposed a hypothetical schema of interactions of the immune system and proteolytic processes involved with the pathogenesis of abdominal aortic aneurysms. It is suggested that initial insults to the matrix may result in degradation of some structural proteins which potentially leads to weakening of the aortic matrix, and products of the degradation trigger further inflammation. According to this hypothesis, an immune response would intensify the degradation of the extracellular matrix, due to increased production of proteases and cytokines. This system would continue to propagate itself without a negative feedback loop such as seen in normal biologic systems.

Clinical manifestations of aneurysms of the thoracic aorta are due to compression, distortion, or erosion of surrounding structures. The most common symptom is pain, which is insidious in an enlarging artery and may be described as boring and deep. Increases in pain intensity may provide a clue to an impending rupture.

Aneurysms of the transverse aortic arch are less common than those found in other sites. Since the innominate and carotid arteries arise from the transverse arch, the consequences of these aneurysms are alarming. Also, the arch is contiguous with other vital structures including the superior vena cava, pulmonary artery, trachea, bronchi, lung, and left recurrent laryngeal nerve, and this makes this aneurysm formidable. Symptoms may include dyspnea, stridor, hoarseness, hemoptysis, cough or chest pain (Cohen, 1996).

Most aneurysms of the descending thoracic aorta occur between the origin of the left subclavian artery and the diaphragm. The most common cause of this type of aneurysm is atherosclerosis, although age, hypertension and smoking are also contributors. In aneurysms of the descending aorta, distortion of the architecture in the area distal to the left subclavian artery, results in sufficient turbulence to cause elastic tissue degeneration, accelerated atherosclerosis and localized dilatation (Cohen, 1996).

Descending thoracic aneurysms are mostly atherosclerotic in origin and occur in older men. They are not common, and involve the celiac, superior mesenteric, and renal arteries. These aneurysms generate intrascapular pain; stretching of the left recurrent laryngeal nerve may produce hoarseness; leakage into the left lung may lead to hemoptysis. Thoracic aortic aneurysms lead to fatality by rupture but are rarely complicated by thrombosis or embolism (Cohen, 1996).

The most common type of aneurysm is the abdominal aortic aneurysm and is frequently seen in men over 60 with ratios of 6:1 seen in men:women. Almost all of these aneurysms are below the renal arteries. Most are atherosclerotic in origin although trauma, infection, and arteritis make up a small fraction. Fortunately, they are easily accessible for physical examination. Rupture of these aneurysms is the greatest threat to the patient and may lead to a rapid demise due to shock and hypotension. A warning to impending rupture is pain in the lower back, which signifies enlargement. Almost all of these types of aneurysms are lined with a clot or have ulcerated plaques. Embolization of atherothrombotic material may lead to symptoms ranging from digital infarction to anuria from a shower of emboli to the kidneys (Cohen, 1996).

Since abdominal aortic aneurysms are the most common type of aneurysm they will be discussed. Physical examination is an important tool for diagnosis and has an accuracy of 30 to 90 percent. The aorta is palpated during exhalation. A pulsatile mass left of midline, between the xyphoid process and the umbilicus, is highly indicative of an abdominal aortic aneurysm. This type of aneurysm has been diagnosed using a plain radiograph, B-mode ultrasound examination, computed tomographic (CT) scan, CT angiogram, magnetic resonance imaging (MRI) and angiography (Santilli et al., 1997).

Treatment of an abdominal aortic aneurysm is dependent upon its size, and has been correlated with risk of rupture. Mortality from rupture is estimated to be 74 to 90 percent of all cases of abdominal aortic aneurysms and elective surgical repair is the choice treatment for patients with aneurysms greater than 5 cm. Most aneurysms are diagnosed in asymptotic patients and are small in size. The annual risk of rupture for an abdominal aneurysm from 5.0 to 5.7 cm in diameter is 6.6 percent, whereas risk of rupturing an aneurysm 7 cm in diameter is 19 percent. It is recommended that elective repair of asymptomatic abdominal aneurysms be carried out for aneurysms greater than 6 cm. The indications for surgical repair of abdominal aortic aneurysms are to relieve pain, prevent rupture of the aneurysm and prolong patient life. These goals are best met when surgical repair is elective (Santilli, 1997).

It is recommended that elective repair be considered for all low-risk patients with an abdominal aortic aneurysm of greater than 5 cm in diameter and an estimated life expectancy of more than 2 years or small abdominal aneurysm (4-5 cm) with documented enlargement of the aneurysm of more than 0.5 cm in less than six months. Very high risk patients include those with poor left ventricular function, nonreconstructable symptomatic coronary artery disease, or severe chronic pulmonary obstructive disease, and should be monitored until the abdominal aneurysm becomes symptomatic or larger than 7 cm (Santilli, 1997).

In order to treat aneurysms or prevent their rupture, a wide variety of therapeutic agents (with or without a carrier) or polymers alone may be delivered to the external portion of the vessel. The polymer or therapeutic agent/polymer complex would be applied to the external portion of the vessel following diagnosis and either through an invasive surgical procedure or through ultrasound, MRI or CT guidance. The purpose of applying these entities to the outside of the blood vessel is to induce or stimulate the formation of a connective tissue layer which would provide added stability and improve the integrity of the vessel wall, thereby preventing the complications associated with aneurysms.

Particularly preferred therapeutic agents include microtubule stabilizing agents, fibrosis inducers, angiogenic factors, growth factors and cytokines and other factors involved in the wound healing or fibrosis cascade.

### G. Formulation and Administration

As noted above, therapeutic compositions of the present invention may be formulated in a variety of forms (*e.g.*, microspheres, pastes, films or sprays). The polymer alone can be applied in the desired form to the outside surface of a body passageway or cavity. Further, the compositions of the present invention may be formulated to contain one or more therapeutic agent(s), to contain a variety of additional compounds and/or to have certain physical properties (*e.g.*, elasticity, a particular melting point or a specified release rate). Within certain embodiments of the invention, compositions may be combined in order to achieve a desired effect (*e.g.*, several preparations of microspheres may be combined in order to achieve both a quick and a slow or prolonged release of one or more factors).

Therapeutic agents and compositions of the present invention may be administered in combination with pharmaceutically or physiologically acceptable carrier, excipients or diluents. Generally, such carriers should be nontoxic to recipients at the dosages and concentrations employed. Ordinarily, the preparation of such compositions entails combining the therapeutic agent with buffers, antioxidants, such as ascorbic acid, low molecular weight (less than about 10 residues) polypeptides, proteins, amino acids, carbohydrates, including glucose, sucrose or dextrins, chelating agents, such as EDTA, glutathione and other stabilizers and excipients. Neutral buffered saline or saline mixed with nonspecific serum albumin are exemplary appropriate diluents.

As noted above, therapeutic agents, therapeutic compositions, or pharmaceutical compositions provided herein may be prepared for administration by a variety of different routes, including, for example, directly to a body passageway or cavity under direct vision (*e.g.*, at the time of surgery or via endoscopic procedures) or via percutaneous drug delivery to the exterior (adventitial) surface of the body passageway (*e.g.*, peritubular delivery). Other representative routes of administration include gastroscopy, ECRP and colonoscopy which do not require full operating procedures and hospitalization, but may require the presence of medical personnel.

Briefly, peritubular drug delivery involves percutaneous administration of localized (often sustained release) therapeutic formulations using a needle or catheter directed via ultrasound, CT, fluoroscopic, MRI or endoscopic guidance to the disease site. Alternatively, the procedure can be performed intra-operatively under direct vision or with additional imaging guidance. Such a procedure can also be performed in conjunction with endovascular procedures, such as angioplasty, atherectomy or stenting or in association with an operative arterial procedure, such as endarterectomy, vessel or graft repair or graft insertion.

For example, within one embodiment, a polymer (with or without a therapeutic agent, such as paclitaxel) can be wrapped (*i*.*e*., film) around an injured blood vessel (*e*.*g*., following a surgical procedure, such as graft insertion), injected into the vascular wall or applied to the adventitial surface allowing drug concentrations to remain highest in regions where biological activity is most needed. The polymer alone or loaded with a therapeutic agent would stimulate the formation of connective tissue and provide the added strength that the vessel requires so as to prevent postoperative complications, such as the formation of pseudoaneurysms.

Another example, in a patient undergoing balloon angioplasty, a sheath is inserted into the artery that is to be catheterized (*e.g.*, femoral) and through which the guidewire and balloon angioplasty catheter will be introduced. The sheath remains in place throughout the procedure, oftentimes causing injury to the site of puncture. After the removal of the balloon angioplasty hardware, a needle would be inserted through the skin to the catheterization site and a therapeutic agent (*e.g.*, paclitaxel impregnated into a slow release polymer) or a polymer alone would be infiltrated through the needle or catheter in a circumferential manner directly around the catheterization site. This could be performed around any artery, vein or graft, but ideal candidates for this intervention include procedures that require arterial and venous catheterization.

The therapeutic agents, therapeutic compositions and pharmaceutical compositions provided herein may be placed within containers, along with packaging material which provide instructions regarding the use of such materials. Generally, such instructions include a tangible expression describing the reagent concentration, as well as within certain embodiments, relative amounts of excipient ingredients or diluents (*e*.*g*., water, saline or PBS) which may be necessary to reconstitute the pharmaceutical composition.

### EXAMPLES

### EXAMPLE 1

### PROCEDURE FOR PRODUCING FILM

The term film refers to a polymer formed into one of many geometric shapes. The film may be a thin, elastic sheet of polymer or a 2 mm thick disc of polymer. This film is designed to be placed on exposed tissue so that any encapsulated drug is released from the polymer over a long period of time at the tissue site. Films may be made by several processes, including for example, by casting, and by spraying.

In the casting technique, polymer is either melted and poured into a shape or dissolved in dichloromethane and poured into a shape. The polymer then either solidifies as it cools or solidifies as the solvent evaporates, respectively. In the spraying technique, the polymer is dissolved in solvent and sprayed onto glass, as the solvent evaporates the polymer solidifies on the glass. Repeated spraying enables a build up of polymer into a film that can be peeled from the glass.

Reagents and equipment which were utilized within these experiments include a small beaker, Coming hot plate stirrer, casting moulds (*e.g*., 50 ml centrifuge tube caps) and mould holding apparatus, 20 ml glass scintillation vial with cap (Plastic insert type), TLC atomizer, Nitrogen gas tank, Polycaprolactone ("PCL" - mol wt 10,000 to 20,000; Polysciences), Paclitaxel (Sigma 95% purity), Ethanol, "washed" (see previous) Ethylene vinyl acetate ("EVA"), Poly(DL)lactic acid ("PLA" - mol wt 15,000 to 25,000; Polysciences), Dichloromethane (HPLC grade Fisher Scientific).

### Procedure for Producing Films - Solvent Casting

Weigh a known weight of PCL directly into a 20 ml glass scintillation vial and add sufficient DCM to achieve a 10% w/v solution. Cap the vial and mix the solution. Add sufficient paclitaxel to the solution to achieve the desired final paclitaxel concentration. Use hand shaking or vortexing to dissolve the paclitaxel in the solution. Let the solution sit for one hour (to diminish the presence of air bubbles) and then pour it slowly into a mould. The mould used is based on the shape required. Place the mould in the fume hood overnight. This will allow the DCM to evaporate. Either leave the film in the mould to store it or peel it out and store it in a sealed container.

### EXAMPLE 2

### THERAPEUTIC AGENT-LOADED POLYMERIC FILMS COMPOSED OF ETHYLENE VINYL ACETATE AND A SURFACTANT

Two types of films were investigated within this example: pure EVA films loaded with paclitaxel and EVA/surfactant blend films loaded with paclitaxel.

The surfactants being examined are two hydrophobic surfactants (Span 80 and Pluronic L101) and one hydrophilic surfactant (Pluronic F127). The Pluronic surfactants were themselves polymers which was an attractive property since they can be blended with EVA to optimize various drug delivery properties. Span 80 is a smaller molecule which disperses in the polymer matrix, and does not form a blend.

Surfactants were useful in modulating the release rates of paclitaxel from films and optimizing certain physical parameters of the films. One aspect of the surfactant blend films which indicated that drug release rates can be controlled was the ability to vary the rate and extent to which the compound swelled in water. Diffusion of water into a polymer-drug matrix was critical to the release of drug from the carrier. Figures 1C and 1D shows the degree of swelling of the films as the level of surfactant in the blend was altered. Pure EVA films did not swell to any significant extent in over 2 months. However, by increasing the level of surfactant added to the EVA it was possible to increase the degree of swelling of the compound, and by increasing hydrophilicity swelling was increased.

Results of experiments with these films are shown below in Figures 1A-1E. Briefly, Figure 1A shows paclitaxel release (in mg) over time from pure EVA films. Figure 1B shows the percentage of drug remaining for the same films. As can be seen from these two figures, as paclitaxel loading increased (*i.e.*, percentage of paclitaxel by weight increased), drug release rates increased, showing the expected concentration dependence. As paclitaxel loading was increased, the percent paclitaxel remaining in the film also increased, indicating that higher loading may be more attractive for long-term release formulations.

Physical strength and elasticity of the films was assessed and is presented in Figure 1E. Briefly, Figure 1E shows stress/strain curves for pure EVA and EVA/surfactant blend films. This crude measurement of stress demonstrated that the elasticity of films was increased with the addition of Pluronic F127, and that the tensile strength (stress on breaking) was increased in a concentration dependent manner with the addition of Pluronic F127. Elasticity and strength are important considerations in designing a film which must be manipulated for particular clinical applications without causing permanent deformation of the compound.

The above data demonstrates the ability of certain surfactant additives to control drug release rates and to alter the physical characteristics of the vehicle.

### EXAMPLE 3

### PACLITAXEL-LOADED POLY(ETHYLENE VINYL ACETATE) FILMS IN A VASCULAR WOUND HEALING RAT MODEL

Wistar rats weighing 250 g to 350 g were anesthetized with halothane (5% induction and 1.5% maintenance). The abdominal aorta was exposed below the renal arteries and blood flow in the aorta was interrupted with two vascular clamps. A 1 cm long arteriotomy was made between the clamps and was immediately repaired with 10-0 non-absorbable sutures. Blood flow in the aorta was restored and the injured aortic segment was treated with 20% paclitaxel-loaded EVA film or 5% paclitaxel-loaded EVA film. In a control group of animals, the wound was left untreated. The abdominal cavity was closed. After 3 days, 7 days, 14 days, 6 weeks or 6 months, the animals were sacrificed and a cannula was introduced in the lower abdominal aorta towards the wound. A ligature was placed around the infrarenal aorta above the wound. Saline was infused through in the cannula with increasing pressure until the wound began leaking. The leaking pressure of the wound was determined in 5 animals in each group. In addition, two animals in each group were injured and treated, but were not subjected to the burst pressure measurement to preserve the cellular structure of their aorta. In these animals, the aorta was removed and processed for histology. Cross-sections of the aorta were cut at the level of the wound and in the intact aorta for comparison. Sections were stained with hematoxylin and eosin and Movat's stains and the effect of paclitaxel on vascular wound healing was assessed.

### Results:

Burst pressure in the different groups is presented in Figure 2. Paclitaxel-loaded EVA films had no effect on vascular wound strength 3 days and 7 days after surgery and treatment, and, in fact, increased wound strength at 14 days after surgery. Animals treated for 6 weeks or 6 months with paclitaxel-loaded EVA films exhibited the same increase in vascular wound strength as animals treated for 2 weeks.

Histology revealed the presence of a periadventitial acellular layer of fibrin in of animals treated with paclitaxel-loaded EVA films for 2 weeks, 6 weeks and 6 months (Figure 3B). This layer was most likely responsible for the increase in vascular wound strength observed. Histology also showed that the vascular wounds healed normally after treatment with periadventitial paclitaxel (Figure 4). Collagen deposition at the site of injury was not affected by the treatment.

### Conclusion:

Periadventitial paclitaxel slowly released from EVA films did not affect vascular wound healing and it increased vessel strength through the formation of a periadventitial fibrin layer. These results suggest that this technology can be applied to the site of vascular surgery to impart added wound strength.

### EXAMPLE 4

### POLY(ETHYLENE VINYL ACETATE) FILMS IN A VASCULAR WOUND HEALING RAT MODEL

Wistar rats weighing 250 g to 350 g were anesthetized with halothane (5% induction and 1.5% maintenance). The abdominal aorta was exposed below the renal arteries and blood flow in the aorta was interrupted with two vascular clamps. A 1 cm long arteriotomy was made between the clamps and was immediately repaired with 10-0 non-absorbable sutures. Blood flow in the aorta was restored and the injured aortic segment was wrapped with an EVA film. In a second group of animals, the wound was left untreated. The abdominal cavity was closed. After 3 days, 7 days, 14 days, 6 weeks or 6 months, the animals were sacrificed and a cannula was introduced in the lower abdominal aorta towards the wound. A ligature was placed around the infrarenal aorta above the wound. Saline was infused through in the cannula with increasing pressure until the wound began leaking. The leaking pressure of the wound was determined in 5 animals in each group. In addition, two animals in each group were injured and treated, but were not subjected to the burst pressure measurement to preserve the cellular structure of their aorta. In these animals, the aorta was removed and processed for histology. Cross-sections of the aorta were cut at the level of the wound and in the intact aorta for comparison. Sections were stained with hematoxylin and eosin and Movat's stains and the effect of the EVA film on vascular wound strength was assessed.

### Results:

Burst pressure in the different groups is presented in Figure 2. Control EVA films devoid of paclitaxel had no effect on vascular wound strength 3 days and 7 days after surgery and treatment, but increased wound strength at 14 days after surgery. Wound strength returned to normal values (*i.e.*, values in injured, untreated animals) at 6 weeks and 6 months.

Histology revealed the presence of a periadventitial capsule of collagen and proteoglycan around the aorta in animals treated with control EVA films for 14 days (Figure 3A). This layer was most likely responsible for the increase in vascular wound strength observed. Collagen deposition at the site of injury was not affected by the treatment.

### Conclusion:

Periadventitial EVA films did not affect vascular wound healing and, in fact, increased vessel strength through the formation of a periadventitial fibrin layer. These results suggest that this technology can be safely applied vascular surgery sites to impart added strength to the healing wound.

### EXAMPLE 5

### CAMPTOTHECIN-LOADED POLY(ETHYLENE VINYL ACETATE) FILMS IN A VASCULAR WOUND HEALING RAT MODEL

Four Wistar rats weighing 250 g to 350 g are anesthetized with halothane (5% induction and 1.5% maintenance). The abdominal aorta is exposed below the renal arteries and blood flow in the aorta interrupted with two vascular clamps. A 1 cm long arteriotomy is made between the clamps and immediately repaired with 10-0 non-absorbable sutures. Blood flow in the aorta is restored and the injured aortic segment treated with 10% camptothecin-loaded EVA film or control EVA film devoid of drug. In a third group of animals, the wound is left untreated. The abdominal cavity is closed. After 14 days, the animals are sacrificed and a cannula introduced in the lower abdominal aorta toward the wound. A ligature is placed around the infrarenal aorta above the wound. Saline is infused through the cannula with increasing pressure until the wound begins to leak. The leaking pressure of the wound is determined. The aorta is removed and processed for histology. Cross-sections of the aorta are cut at the level of the wound. Sections are stained with hematoxylin and eosin and Movat's stains and the effect of camptothecin on vascular wound healing is assessed.

### Results:

The wound of three-quarters of the animals treated with 10% camptothecin EVA films exhibited a 4 fold increase in strength compared with animals treated with control EVA film and untreated animals. The 3 animals with high wound strength displayed a periadventitial fibrin capsule on histopathology examination. The fourth animal with a low wound strength did not possess a complete capsule.

### Conclusion:

Periadventitial camptothecin released from EVA films increases vessel strength by inducing the formation of a periadventitial fibrin capsule. These results suggest that this technology can be applied to the site of vascular injury to impart added wound strength.

## Claims

1. Use of a factor that induces fibrosis for the manufacture of a medicament for improving or increasing body passageway or cavity integrity, comprising delivering said factor to a non-luminal surface of said body passageway or cavity.

2. Use according to Claim 1 wherein the factor is delivered via the adventia.

3. The use according to Claim 1 or 2 wherein said. therapeutic agent further comprises a polymeric carrier.

4. The use according to Claim 3 when dependent upon Claim 1 wherein said polymeric carrier is formed into a film

5. The use according to Claim 3 when dependent on Claim 1 wherein said polymeric carrier is formed into a wrap.

6. The use according to Claim 3 wherein said polymeric carrier is formed into a gel.

7. The use according to Claim 3 wherein said polymeric carrier is formed into a foam.

8. The use according to Claim 3 wherein said polymeric carrier is formed into a mold.

9. The use according to Claim 3 wherein said polymeric carrier is formed into microspheres having an average size of between 0.5 and 200 µm.

10. The use according to Claim 3 wherein said polymeric carrier is poly(ethylene vinyl acetate).

11. The use according to Claim 3 wherein said polymeric carrier is a copolymer of poly(lactic acid) and poly(glycolic acid).

12. The use according to Claim 3 wherein said polymeric carrier is poly(caprolactone).

13. The use according to Claim 3 wherein said polymeric carrier is poly(lactic acid).

14. The use according to Claim 3 wherein said polymeric carrier is a copolymer of poly(lactic acid) and poly(caprolactone).

15. The use according to Claim 3 wherein said polymeric carrier is poly(urethane).

16. The use according to Claim 3 wherein said polymeric carrier is hyaluronic acid.

17. The use according to Claim 3 wherein said polymeric carrier is chitosan.

18. The use according to Claim 3 wherein said polymeric carrier is silicone.

19. The use according to Claim 3 wherein said polymeric carrier is poly(hydroxyethylmethacrylate).

20. The use according to Claim 1 wherein the factor is camptothecin, or an analogue or derivative thereof.

21. The use according to Claim 1 wherein said body passageway is selected from the group consisting of arteries, veins, the heart, the esophagus, the stomach, the duodenum, the small intestine, the large intestine, biliary tracts, the ureter, the bladder, the urethra, lacrimal ducts, the trachea, bronchi, bronchiole, nasal airways, eustachian tubes, the external auditory canal, vas deferens and fallopian tubes.

22. The use according to Claim 1 wherein said cavity is selected from the group consisting of the abdominal cavity, the buccal cavity, the peritoneal cavity, the pericardial cavity, the pelvic cavity, perivisceral cavity, pleura cavity and uterine cavity.

23. The use according to Claim 1 wherein said therapeutic agent is delivered to a body passageway or cavity by direct injection *via* an outer wall of the body passageway or cavity into the adventia.

24. The use according to Claim 1 wherein said body passageway is an artery or vein.

25. Use according to any preceding claim wherein the medicament is for the treatment or prevention of iatrogenic complications of arterial and venous catheterization.

26. Use according to any preceding claim wherein the medicament is for the treatment or prevention of pseudoaneurysms.

27. Use according to any preceding claim wherein the medicament is for the treatment or prevention of aneurysms.

28. Use according to any preceding claim wherein the medicament is for the treatment or prevention of cardiac rupture and dissection.

29. Use according to any preceding claim wherein the medicament is for the treatment or prevention of vascular dissection.

30. Use according to any preceding claim wherein the medicament is for the treatment or prevention of periprosthetic leaks and cardiac valve dehiscence.

31. Use according to any preceding claim wherein the medicament is for the treatment or prevention of gastrointestinal passageway rupture and dissection.

32. Use according to any preceding claim wherein the medicament is for the treatment or prevention of complications associated with vascular surgery.

## Revendications

1. Utilisation d'un facteur induisant la fibrose pour la fabrication d'un médicament afin d'améliorer ou d'augmenter l'intégrité d'une voie de passage ou d'une cavité du corps, comportant la délivrance du dit facteur à une surface non luminale des dites voies de passages et cavités du corps.

2. L'utilisation conformément à la revendication 1 dans laquelle le facteur est délivré au travers de l'adventice.

3. L'utilisation conformément à la revendication 1 ou 2 dans laquelle ledit agent thérapeutique comprend de plus un porteur polymérique.

4. L'utilisation conformément à la revendication 3, alors que dépendante de la revendication 1, dans laquelle ledit porteur polymérique est formé en un film.

5. L'utilisation conformément à la revendication 3 alors que dépendante de la revendication 1, dans laquelle le dit porteur polymérique est formé en feuillets.

6. L'utilisation conformément à la revendication 3 dans laquelle ledit porteur polymérique est formé en un gel.

7. L'utilisation conformément à la revendication 3 dans lequel ledit porteur polymérique est formé en une mousse.

8. L'utilisation conformément à la revendication 3 dans laquelle ledit porteur polymérique est formé en un moule.

9. L'utilisation conformément à la revendication 3 dans laquelle ledit porteur polymérique est formé dans des microsphères ayant une taille moyenne comprise entre 0,5 et 200 µm.

10. L'utilisation conformément à la revendication 3 dans laquelle ledit porteur polymérique est un poly (éthylène vinyle acétate).

11. L'utilisation conformément à la revendication 3 dans laquelle ledit porteur polymérique est un copolymère du poly (acide lactique) et poly (acide glycolique).

12. L'utilisation conformément à la revendication 3 dans laquelle ledit porteur polymérique est un poly (caprolactone).

13. L'utilisation conformément à la revendication 3 dans laquelle ledit porteur polymérique est poly (acide lactique).

14. L'utilisation conformément à la revendication 3 dans laquelle ledit porteur polymérique est un copolymère du poly (acide lactique) et poly (caprolactone).

15. L'utilisation conformément à la revendication 3 dans laquelle ledit porteur polymérique est poly (uréthane).

16. L'utilisation conformément à la revendication 3 dans laquelle ledit porteur polymérique est un acide hyaluronic.

17. L'utilisation conformément à la revendication 3 dans laquelle ledit porteur polymérique est un chitosane.

18. L'utilisation conformément à la revendication 3 dans laquelle ledit porteur polymérique est une silicone.

19. L'utilisation conformément à la revendication 3 dans laquelle ledit porteur polymérique est un poly (hydroxyéthylméthacrylate).

20. L'utilisation conformément à la revendication 1 dans laquelle le facteur est une camptothécine, ou un analogue ou un dérivé de celui-ci.

21. L'utilisation conformément à la revendication 1 dans laquelle ladite voie de passage est sélectionnée depuis un groupe constitué d'artères, de veines, du coeur, de l'oesophage, de l'estomac, du duodénum, de l'intestin grêle, du gros intestin, des voies biliaires, l'uretères, la vessie, l'urètre, les voies lacrymales, la trachée, les bronches, les bronchioles, voies aériennes nasales, les trompes d'eustaches, le canal externe de l'oreille, le canal déférent et les trompes de fallope.

22. L'utilisation conformément à la revendication 1 dans laquelle ladite cavité est sélectionnée depuis le groupe constitué par la cavité abdominale, la cavité buccale, la cavité péritonéale, la cavité péricardique, la cavité pelvienne, la cavité péri-viscérale, la cavité pleurale et la cavité utérine.

23. L'utilisation conformément à la revendication 1 dans laquelle ledit agent thérapeutique est administré dans une voie de passage ou d'une cavité anatomique par injection directe au travers d'un mur exteme de la voie de passage ou de la cavité anatomique dans l'adventice.

24. L'utilisation conformément à la revendication 1 dans laquelle ladite voie de passage est une artère ou une veine.

25. L'utilisation conformément à l'une quelconque des revendications précédentes dans laquelle le médicament est destiné au traitement ou à la prévention des complications iatrogéniques d'une cathétérisation veineuse ou artérielle.

26. L'utilisation conformément à l'une quelconque des revendications précédentes dans laquelle le médicament est destiné au traitement ou à la prévention des pseudo anévrismes.

27. L'utilisation conformément à l'une quelconque des revendications précédentes dans laquelle le médicament est destiner au traitement ou à la prévention des anévrismes.

28. L'utilisation conformément à l'une quelconque des revendications précédentes dans laquelle le médicament est destiné au traitement ou à la prévention des dissections et ruptures cardiaque.

29. L'utilisation conformément à l'une quelconque des revendications précédentes dans laquelle le médicament est destiné au traitement ou à la prévention des dissections vasculaires.

30. L'utilisation conformément à l'une quelconque des revendications précédentes dans laquelle le médicament est destiné au traitement ou à la prévention des fuites périprothétiques et des déhiscences des valves cardiaques.

31. L'utilisation conformément à l'une quelconque des revendications précédentes dans laquelle le médicament est destiné au traitement ou à la prévention de la rupture et de la dissection des voies de passages gastrointestinates.

32. L'utilisation conformément à l'une quelconque des revendications précédentes dans laquelle le médicament est destiné au traitement ou à la prévention des complications associées à la chirurgie vasculaire.

## Patentansprüche

1. Verwendung eines Faktors, der Fibrose induziert, zur Herstellung eines Medikaments zur Verbesserung und Erhöhung der Integrität eines Körperpassageweges oder einer Körperhöhle, die die Zuführung besagten Faktors zu einer nichtluminalen Oberfläche von besagtem Körperpassageweg oder Körperhöhle umfasst.

2. Verwendung gemäß Anspruch 1, worin der Faktor über die Adventia zugeführt wird.

3. Die Verwendung gemäß Anspruch 1 oder 2, worin besagtes therapeutisches Agens zusätzlich ein polymeres Trägermittel umfasst

4. Die Verwendung gemäß Anspruch 3 in Abhängigkeit von Anspruch 1, worin besagtes polymeres Trägermittel als ein Film ausgeformt ist

5. Die Verwendung gemäß Anspruch 3 in Abhängigkeit von Anspruch 1, worin besagtes polymeres Trägermittel als Wickel ausgebildet ist.

6. Die Verwendung gemäß Anspruch 3, worin besagtes polymeres Trägermittel als ein Gel ausgeformt ist.

7. Die Verwendung gemäß Anspruch 3, worin besagtes polymeres Trägermittel als ein Schaum ausgebildet ist.

8. Die Verwendung gemäß Anspruch 3, worin besagtes polymeres Trägermittel als eine Gussform ausgebildet ist.

9. Die Verwendung gemäß Anspruch 3, worin besagtes polymeres Trägermittel als Mikrosphären mit einer durchschnittlichen Größe von 0,5 bis 200 µm ausgebildet ist.

10. Die Verwendung gemäß Anspruch 3, worin besagtes polymeres Trägermittel Poly(ethylenvinylacetat) ist.

11. Die Verwendung gemäß Anspruch 3, worin besagtes polymeres Trägermittel ein Copoylmer aus Poly(milchsäure) und Poly(glykolsäure) ist.

12. Die Verwendung gemäß Anspruch 3, worin besagtes polymeres Trägermittel Poly(caprolacton) ist.

13. Die Verwendung gemäß Anspruch 3, worin besagtes polymeres Trägermittel Poly(milchsäure) ist.

14. Die Verwendung gemäß Anspruch 3, worin besagtes polymeres Trägermittel ein Copolymer aus Poly(milchsäure) und Poly(caprolacton) ist.

15. Die Verwendung gemäß Anspruch 3, worin besagtes polymeres Trägermittel Poly(urethan) ist.

16. Die Verwendung gemäß Anspruch 3, worin besagtes polymeres Trägermittel Hyaluronsäure ist.

17. Die Verwendung gemäß Anspruch 3, worin besagtes polymeres Trägermittel Chitosan ist.

18. Die Verwendung gemäß Anspruch 3, worin besagtes polymeres Trägermittel Silikon ist.

19. Die Verwendung gemäß Anspruch 3, worin besagtes polymeres Trägermittel Poly(hydroxyethylmethacrylat) ist.

20. Die Verwendung gemäß Anspruch 1, worin der Faktor Camptothecin oder ein Analogon oder ein Derivat davon ist.

21. Die Verwendung gemäß Anspruch 1, worin besagter Körperpassageweg aus der Gruppe ausgewählt ist, die aus Arterien, Venen, dem Herz, der Speiseröhre, dem Magen, dem Zwölf-Fingerdarm, dem Dünndarm, dem Dickdarm, den Gallenwegen, dem Harnleiter, der Blase, der Harnröhre, den Tränendrüsen, der Luftröhre, den Bronchen, den Bronchiolen, den Nasenatemwegen, den eustachischen Röhren, dem externen Gehörgang, dem Vas deferens und den Eileitern besteht.

22. Die Verwendung gemäß Anspruch 1, worin besagte Höhle aus der Gruppe ausgewählt ist, die aus der Bauchhöhle, der Mundhöhle, der peritonealen Höhle, der perikardialen Höhle, der Beckenhöhle, der perivisceralen Höhle, der Pleurahöhle und der Gebährmuttefiöhle besteht.

23. Die Verwendung gemäß Anspruch 1, worin besagtes therapeutisches Agens einem Körperpassageweg oder einer Körperhöhle durch direkte Injektion über eine äußere Wand des Körperpassageweges oder der Körperhöhle in die Adventia zugeführt wird.

24. Die Verwendung gemäß Anspruch 1, worin besagter Körperpassageweg eine Arterie oder Vene ist.

25. Verwendung gemäß einem der vorangegangenen Ansprüche, worin das Medikament zur Behandlung oder Verhinderung iatrogener Komplikationen bei arterieller und venöser Katheterisierung bestimmt ist.

26. Verwendung gemäß einem der vorangegangenen Ansprüche, worin das Medikament zur Behandlung oder Verhinderung von Pseudoaneurysmen bestimmt ist.

27. Verwendung gemäß einem der vorangegangenen Ansprüche, worin das Medikament zur Behandlung oder Verhinderung von Aneurysmen bestimmt ist.

28. Verwendung gemäß einem der vorangegangenen Ansprüche, worin das Medikament zur Behandlung oder Verhinderung zur Behandlung eines Herzrisses und Sektion bestimmt ist.

29. Verwendung gemäß einem der vorangegangenen Ansprüche, worin das Medikament zur Behandlung oder Verhinderung vaskulärer Risse bestimmt ist.

30. Verwendung gemäß einem der vorangegangenen Ansprüche, worin das Medikament zur Behandlung oder Verhinderung von periprosthestischen Undichtigkeiten und Herzklappendehizenz bestimmt ist.

31. Verwendung gemäß einem der vorangegangenen Ansprüche, worin das Medikament zur Behandlung oder Verhinderung von gastrointestinalen Passageweg- rissen und Sektion bestimmt ist.

32. Verwendung gemäß einem der vorangegangenen Ansprüche, worin das Medikament zur Behandlung oder Verhinderung von Komplikationen bestimmt ist, die mit vaskulärer Chirurgie assoziiert sind.
